# EUROPEAN PATENT APPLICATION

(11) **EP 3 858 999 A1**
(43) Date of publication of application: **04.08.2021**
(21) Application number: 20154496.2
(22) Date of filing: 30.01.2020
(51) Int. Cl.: C12N 15/90, C12N 15/10

(54) **SAFE HARBOR LOCI**

(71) Applicant: Aelian Biotechnology GmbH, 1030 Vienna (AT)
(72) Inventor: JUDE, Julian, 1140 Vienna (AT); VASILYEV, Anatoly, 1020 Vienna (AT); KREJCI, Adam, 1020 Vienna (AT); BUERCKSTUEMMER, Tilmann, 1060 Vienna (AT)
(74) Representative: Sagittarius IP

(57) **Abstract**

An isolated mammalian host cell comprising a heterologous gene of interest (GOI) chromosomally integrated at a target site within an intergenic region between a pair of adjacent essential genes.

SEQUENCE LISTING

<110>  Aelian Biotechnology GmbH

<120>  SAFE HARBOR LOCI

<130>  AE002EP

<160>  30

<170>  PatentIn version 3.5

<210>  1
<211>  52
<212>  DNA
<213>  Homo sapiens

<400>  1
aagtgctgcc cggcgctccg gaagcgattc atcgggccgc gagcgccctc cc          52

<210>  2
<211>  119
<212>  DNA
<213>  Homo sapiens

<400>  2
cagcctctcg gccccgcctc gatttttagc tttataggaa tgctgttgct ttaaatccga      60

aatcccgtgc cggtatcaac tctcgcgatc tccgaggccg catacatatt acccacaat      119

<210>  3
<211>  125
<212>  DNA
<213>  Homo sapiens

<400>  3
tggcgcgctg cgatgacgtc ggcggcacgc ctgcgactcg ggctccgcgc aaaagatggg      60

gttggggtac ggcgcgtaga gatgacgtcg ggttctacgc gcagtggtga cgtcacggga     120

gcgcc                                                                 125

<210>  4
<211>  141
<212>  DNA
<213>  Homo sapiens

<400>  4
cttgccagcc ccggacttct gcgcgcgctg catgcccatt ggatgtgctc attcccaccc      60

cagccaatcc cgaaactcgc tcggacgctg acagaagact gcgccgctgc tttgggattg     120

gtagctgagt tttgtgtcgc g                                               141

<210>  5
<211>  195
<212>  DNA
<213>  Homo sapiens

<400>  5
agaaggtgga aggagaaaag aaaacttcgc gccaatcggc gtggccccgc ccccagaccg      60

tcccttcgtt gcgacaccaa ctagctcgcc ccgcccactc cgtacaccta agaggggcgc      120

atgacgtaca tccgctaaca tgcagccgcc atcattgatt cgataggcga cgcggggagc      180

ggaagccctt tacac                                                      195


<210>  6
<211>  239
<212>  DNA
<213>  Homo sapiens

<400>  6
agctcccact gccgctcagc gccgatgcgc cgcccgcctc gagctcacat tggtcctggc      60

agccttcccg ccacaccaac caaccaatag acagggcgat tctgcgctcc cggccctgct      120

gcaggctgtc tcgcacttgt cattggtcac tgcagccgcc ccaccccccc cggcgcgcca      180

gtggctgggc ggcctcgctg gggcgggccg cagttcctgc gcgtgcgcgc ttggcctcc      239


<210>  7
<211>  341
<212>  DNA
<213>  Homo sapiens

<400>  7
tcctgaaaga cacgtcagcc ggaagcaaga cacgggcacg ctaggaaatg tagtttactt      60

tttccgagcg gcggcgaagt aagccaatgg gaagctccac aataatatgc aagggggcgg      120

aaggcgtaag tgcgtcacgg agagcatctc gggaattgta gtgtgtttct gcaagccaat      180

gggatccggg gaaacatgat aaggatgcgg tcgaggctgg gaataggctg agggagctgg      240

agagggtggg accacgcagg aggcagatcc aataaacagg aagatttttc tcgtgacgtc      300

gtcggcgcgc gccggaagcg cggatcacac gggcccctac a                         341


<210>  8
<211>  343
<212>  DNA
<213>  Homo sapiens

<400>  8
gtatgcagct aactacttcc gcttccgctt gcgtcccctg cgtccctggc tcgaggtttt      60

ccgccatttt gagtgtggcc ctagattgtt ctcaatactc tgtccttggg ttttgtaagt      120

tgaaagttga gactaggaag gcatatcaca agaactataa acagagtttc aatgaggcaa      180

cactgaggag gagttctact ccttcctcat ccccttccaa agcccctgaa ctcgagtagc      240

cgcccctccc atcatggttc ccttagtgtg gtctcgccgc acaccccgcc cattgacccg      300

gaattcttct ctgcctggtc ttcaggtccc aatcctccgc ttc                       343

<210> 9
<211> 371
<212> DNA
<213> Homo sapiens

<400> 9
tccggaaata cgtaaaaaca gagcgccggg tccacgcctt ccccaaggaa tggggcgtgt 60

ctcggcaccc ctcggtgacg cctccactga catcatctgc ggattcccgc cccgaggttt 120

ctaaatccag acattcccgt ttggctgagc actctaggcc tacatccatg aagctaggag 180

agcgacactc aaagactgca ctattgagag aagctaacgt taaaggcagt gaatatattc 240

gggagtccag ctccggaacc cgggagctct tttagtggga ggggcggcgc tgatggcgct 300

tctggcctcc gaatgctagg gggcgctgtg atcgccgggc ggcctcttgg gcgctgggag 360

tccaccgcgc a 371

<210> 10
<211> 441
<212> DNA
<213> Homo sapiens

<400> 10
cgttagaatt caaaggttct tctttttaaa taggagttaa accctctagc tagaaagccg 60

attggcctgc gctcgagcga ggctgccttg tgattggttg acaacaaacc cgacgccttg 120

agtcgcgttt aagggcgcac accgcgcgca ggcgcacttg ctccctaaga acccggaagt 180

ggtggagcca acggccagcc agacctgtgg cagtcccagc cccagggtgc tctccgcggt 240

cggccattta tcggtgaagc aacagcccct tgacgtcaag cttgacgcat tggagataaa 300

gtccaaggag gtgaactaac ctttgaacgt gccagtaagt aagatggcga gaaaagcaag 360

agagagtggg accgatcgat agcgcagcgg tcggcttggc gccctttcag cgtgcgcagt 420

gaacgtgcgc tcggagcggt a 441

<210> 11
<211> 527
<212> DNA
<213> Homo sapiens

<400> 11
gctcccctca ctccagttgc tcagcccgcc gctgagcaca gccaatccgc ggccgtccac 60

tcactgtttc ggccaatcac cgagcagacg gcccggaagc tcgagaggct cgtgccaggt 120

cgggggcgga aggcgaggcc agcccaccaa gcaggggcgt caccaaggga aagccaatca 180

ccattatttc tgaagcccaa gggggcggga cgagaagtca acataatggt ggtgctgctg 240

gaaatctagc acctggggag agcctgagac ttgtgtatct aattgcctat tggacacctc 300

catctgggta aacccaggca tccagaactt tccctcaaga cttacctccc tcagtgcagg 360

tcgggctggc ctcacgcatt tcgcttggat gcctaaatct gtctccacgt ggggccggtc 420

gataaaacaa cacttcccag aatacgctgc gatccattcg cggtctgcac ccggaaagta 480

cacctcccag gggtctgtgc gcgcctccag ggaacgggtc ttgtggc 527

<210> 12
<211> 546
<212> DNA
<213> Homo sapiens

<400> 12
gtcagctggt tgtgtcactt gacccctctt gttgctaaga ggcagcaggg acacccattt 60

tcacttccgc ttccggttgt cacaggcgac gaagggggag gacagaacag aggcgttgcc 120

cggatgtcga ggcgcgcgca ccgcggaacc acccctcaat tccggttcgc ctcccaaccc 180

ctccccatcc agcttccctg cggctgcgca atacgcggca cgcgccaggc caaagactag 240

gagcgaagcg tgcacgcctt gtctttctcc acctttcgtc tttggtcaca gcgccatctc 300

atggttagct gaaactttga aagaggattc attcttcaga ggcaaggaat cggagtccta 360

tgtctttctc gcgccttgaa ggttacttag ctaaagctaa gatagcgaga aacagtactt 420

ttcaggtcct tccatgatct acattcgtcc ttgttctcgg aggacccaga ctctatggtt 480

aatgatgaag aggggcctct attcgttcag cgcttgcgca tgcgaggagg ttccgcatgc 540

gcggtg 546

<210> 13
<211> 611
<212> DNA
<213> Homo sapiens

<400> 13
ccgcgcttgc tccgccgcgc tacaggtcgc gggaggggcc aggcctaggt gcttcgccaa 60

aagagggcgg agctgcgcac acgtgctggc tccccgccgc ctcgggctcg gctctctccg 120

cgcagaggtt tcatggcttg cttcaatctt ttgatatctt agagtaattg cttccaattt 180

ttgggcatcg ccgcctcagt ttacagaata gtatggatct ggtgccagag ccgggaaaac 240

gatgcgcatc gtaaaccgca aaacactgag catccaggac agaagccaag gcgcttggct 300

tggttgaagg ccagtgcgca tgcgcgagtt tacccttcgc ggactgggcg agctcttgcc 360

actcctacct ccgggcttca gtcttcgccg cctgttctgg tcctttcccg ggccgctatt 420

ggaagaaccg tgaactatgg aaggcccctc tggattggct gggctggccc aagagtaagg 480

agagccctct tcctggcggt ggggaaggga cggcggcgat tggcggacgc tcctggcagg 540

agagcgcgga ttggtcaggc acggagcagg aggcggggct gatagcccag cagcagcagc 600

ggcggcggcg g 611

<210> 14

<211>  736
<212>  DNA
<213>  Homo sapiens

<400>  14
agccggtacg ccccagaggc tcaccggaag tgccggaccc gagacccgga gggggggtgc      60

ccgggacaaa gcgtcggctg caaaagagaa aaggccaggc gaagacgcta ggctctaaaa     120

caccctcagt agaagcatta aaatgccctt tcgtgattta tttccccttt tataggtatt     180

tttaaacgtt attttaaatt acgtgttgac agtattcccc tccagacgaa tttagtaggg     240

cccattgtcc ttacgactac actacccaca atgcactacc cagactgcct ctgcttcagc     300

catcgaggac tcgggcggaa ctaagctaca aatccttctt caaaccacac caaaaaccta     360

ttgcaaacgt aaatgctgcc ccattgggta tgtgactgag tttaatgtat ggttagaagt     420

atactttccc tgagggttcc cagtgggagg cagtaggctt ctcttcccaa agtaaagatg     480

tccgcccagc gtcgcttttc tgggtaaaag cgagtcagag ttgaaggggg caacagtgtt     540

tgcgttcggc ctcagaggtg acgcttcttt gggggcgggc tggagttttc tggccgtgaa     600

tggcagagcg ctaatggccg cctccaatgt ggcccaatca gaaagaaagg aaggctggga     660

actaagaagc tattggttgg tgatccctgg accaatcgga ggagccgtga tttggcggga     720

gtcttgaccg ccgccg                                                     736

<210>  15
<211>  737
<212>  DNA
<213>  Homo sapiens

<400>  15
   ttcttccttg tcaccctccg cagtttggca ggacagccaa taggcacacg agatcctcca      60
   tcaaggggcg ttcccatctg gggatcccca cgccggcagc accaaggcct ctgggtcagc     120
   ttctttaagg gcaagcgccc ttagagaagc agcacggaag caaccaatca actggtttct     180
   ccaattagcc cccaagtgga tcagcctgct gaactgtcgg ctagggttat aggtggttct     240
   gagtccttcc ggaagctgcc gaatcttaga agtcgcgcca ctgttgtggc gatggaagaa     300
   ggcgaagctc tacagaaacc attttggttg agggaagagt ggttttaaag ttaatttgga     360
   aaggagccgg caagcaacct tcagtcggtg tgcggttttt acagttgaac tgtttccagc     420
   cgctaattat gattgtttta gcggttcgtc atctgaaaac tgcattctgc acggcggtgg     480
   tggggggagg acgaggatgg agaagcctgg gcggacatat ttattagctg cggtcctaag     540
   tcacgtgtga cattctgtat ccagtctatc ggttatccca aggagaacgc cctgccggaa     600
   tggccaatcg ccttgagatg tccccgccct attcgccatc ttaacatagg gcattcccca     660
   cagctgcagg caccagtgaa gcgtctaagg gaccatcttg gttgagggcg gaagtgagga     720
   ctgtgtcgcg ctggagg                                                    737

<210>  16
<211>  813
<212>  DNA
<213>  Homo sapiens

<400>  16
gctgtgacct ttcaccccgc ccctcgcgcg ccggcgcgcc gccgcctcac gcgcagccgc      60

actgctcgcc tccgcgcacc ctgccccctc cactccggaa cctccatctt tctcctggac     120

tgggccttct ggggtcatgc tcctccctcc cacccgctta cgacactgct tgtggggctg     180

gctcggtagg ccctggcggt aaaatccaga tttttccacc cctccctctc ttgtcccata     240

tccgcaagat agtttttctc agccccgttc ttcccagttt gtggtctcca gagtgacctt     300

caaacgctgt tgcctcctag tagggtttta taaagttagt tcttctaaat gtaaagaaat     360

ctgtggaaag ctcaagttta agttaggtgt tttaaaacaa acgtagttaa caatcggcca     420

gatagattgg ctaccctcca gcacgcctca agttttaaga tcacgtgatt ggcccaaacc     480

tcagagcctg gaattttacc ggcggactcc agagggacgg aacgccaccg ccatttcctg     540

tccctcgatt gtggcgtgaa cgtcacttcc ttagcaatgc gctttaactc ttgctgtcca     600

cattttccac attgtacttt ataacatact ctttgtgaaa atttatcttt tatttcttta     660

atccaagaaa attaatattc aagggatccg taaaaattca ggccccccga gatgtagagc     720

gctgggatgg aggaggcagg gcggagctcg acgtcatgtg actggttggg cccgcccccc     780

cccccccccc caacctgtgc agctgaggac ttc                                  813


<210>  17
<211>  1433
<212>  DNA
<213>  Homo sapiens

<400>  17
actctcgggt cttcccgcgg ccgccgaaga cccgctgagg cagaagctgc ccgcggcgcg      60

cgccttatca aaaacccgtt ttcttgccat tgaaagagaa actgggaact tagataacag     120

gaaaatctcc acatacacta aattcacatt attttatacc ctgtaaaagc tcccaccaga     180

tataaaaact cattataatg gcatgcgttc cgtcacaact tcggacagaa gttgggtctt     240

tgggcaggaa gtggaagcca tagaggaagt catggagcga gagtgatgtc tgtagtgaag     300

aatttggcgc catcttgaga aggtcaaagt gattcttcga ttaaatgcct tacttatcgc     360

ctagtgtgac ctggcgcttg ggtttactct ccccagcaac tgcgcctcaa acgcaaagaa     420

aactgcaggt cctcggggaa ggatcgattt caccattcaa gagaaaaatc catttcccag     480

attcctcccc tttatgtaaa cgtctggtac ctagtagacg ttaaatacac gtgagttttc     540

cggttttata agatcacata ctgcaaacca acagtaaatt acattaatac gcatttcttt     600

agaaattctt aattgctagg cactgacata taaattgagt ttgctgttgt tttagagaga 660

aaaatagaca tattcatttt ttttcaatca ggacatggta ttcgtttcct tcttaagctt 720

cccatttttg aggtaggaaa gtattggtgt gtgtctctat ctctaccttg ctccctcatt 780

agcaggctta atgggcagga attatgtctg ttttgctttc cgttggatcc ccgggtctag 840

tagagcgcct tgacacatag taggtactcg acacagatgc tggcttcatg atgaagcaat 900

tgtcctcgaa ttaatacaaa agggaagatt cagactcagc gagccggtac cattcgtcta 960

gggacacaca gccaggaagg aaccgaacca ggattctaac ccaggattac ccaagttcga 1020

attctaattg aacataagtc cttagcgacc ttggacggac ttactaaact gccctgaatc 1080

tcggcttcct tagctgcaaa atagggttag gaaaagtgga ctcggtgctc ctctgcgccg 1140

cattcccgca gcgggatttg tgaaagcgaa cttgagcccc acactgcaga agccctgagg 1200

tcctgtctca accagaatcc agcacccagg acaagccctc cagcgacttc agaccttccc 1260

gctcccgacc ccgcccgccc ccgagaggcg tggtcaaggc ggggccagaa ggcggacgcg 1320

tgcacggagc ggtaaagcgc aggcgcaaag cttcggtgac gtcagagagg cgcgctccca 1380

gctcggagcc gactcgcaga cgcgccccgc ccctcggcgt cgctctggac tgg 1433

<210> 18
<211> 2020
<212> DNA
<213> Homo sapiens

<400> 18
gtctctctgc gcatgctcac acgcgacctc cccacaaagc tactccgcgg gcctgcctgg 60

ctactgagtc gcgaaaatat atcgtagtgc cgcaatgact tctgggtact gtagtccaac 120

atgtggactg gccactcggc ttgaagcttc cttgggattg gatgttggga ccgtccaaac 180

acggcgactt ctcatttgtc agcctgcggc agtttaaatt gggtggcggt ggtggcatgt 240

cctgttagct gtggagtctt ttcctcttat cttgcgcctc cggttgtgcc ttaatgacac 300

ttttgacatt gtgtatcaca accaactggc aaaaactagt aaatcacggg ctcctgacgt 360

ggcagctctg gctaaaatgc aggtggacgt cctaggtgcc cagggagttc gggctcactg 420

gggagtaggg tgaaggaaac cctgaaaccg agaggctgga agcccgactt gcgcagcata 480

aaaacacctg cagggggaaa gcctacatga ggtacgggtt gtcatcccgt ctcatcttgg 540

ccatctgact gatggagatc ctcaggcacc cagcagactg gggtgtcacc cccactaccc 600

actgcccagg gctgacctcg ggcggaagag cgacattcaa atatttgttg catatggagc 660

aacagaccag ggtggaagag agagaaagga ctttccagga aggtccaggc ttgccaggac 720

ttctatgctt ggactgctct gtccccaagc tcgctggtgc ccatcaggag cctgcttggc 780

acctttgctt atttcttcga ttgttcctca actcagaggg ggagaggcaa gattatcact 840

cccatagcct ataacatgca cgcttaaaca caacccctct gacatggctt cagaccgcat 900

ccacaggtga aaagcaccaa atccaaacaa tcaggccaca aacccgtcaa gagctcgccg 960

gtctggccgt ccagtcgccg cccgccttga gactcagcct gagctacagc ttcgaaacta 1020

gcctctagcc ttctgggtct cagcacaact cttgagcaat ccaaccctgt ggccgtgggc 1080

agataccaag atgcacagtt agcacctccg acaggaccgc ggctcggcgg ggctcgccgt 1140

ctacacgccc attcccaggc ccacggccac gacacccaga gctgcctcct gccctgggct 1200

ccctggagcc tggtggccgc ggctcatgct gcagtcaccg tgtggccagc tgccaagcgc 1260

cactacttgc acctgaagtc gcagccccgc gctgcgcacc cagtcgaggc gccaacacgc 1320

gaactgggcg ggagtggccg gcccgcgggg gagcgaggaa cgacagggtt ccccccactc 1380

gctgcccaga ctggccgcgg gatagcgcgc cccccccaaa gcctgcaccc ctgccctgct 1440

cctctatcat tccgtccccc ttgctctgtc aaaccctgcc ggggctgctc tgtctctgcc 1500

gacccgccac ttgcccgaag ttcggcctga ggagcttggg gccctgagga gcgccgacgt 1560

aggcagccag ccagtcaatc agtcagaggg tcacggcggc ccctcggcta ctcagtggca 1620

gccaggccag aaagaagcag ccgccaaccc tggagcagaa tcctacagcc gacctgacct 1680

agtaggcccc cagccctcca gagcttgggg tattgcagag ccgccacaag gcctgccggg 1740

agtagtagtt tgtatagttc gcagccggcc agggcggagc cgggtgggtt ggtggggagg 1800

gcggggaaac aagcctggac cacaactccc aggagtaccc gcgacggcgg cggcctcgct 1860

gtcgcactca ggctataggg ggcgcattgg gcggaagacc ctgggccggc aggaacttgc 1920

ttgtgattgg atgttgtggg acggcggctt ctcattcgtc agcctgtgac tgtggagttt 1980

gaattgggtg gcggttgact gtagagccgc tctctctcac 2020

<210> 19
<211> 4357
<212> DNA
<213> Homo sapiens

<400> 19
cacatctgcg gcatcctctc ggccaccgta gagtgtccca cgtgtccgct tcctcttccg 60

ctactcgccc gcgcgtctgt ctgtcaagag agacttccgg catgcctttt tttttttttt 120

ttttcccttt ccccgcaccg tcacctggtg gcgcttactg gaagccgcac cgaggcgtgt 180

gctgtatttt aaaaatccca tttctggtca atttctccac gtaatcagac acccgcagag 240

aaactgcata taactctttg cctcaaggct tttagacaag cagaggccaa aaacttgcat 300

aaaatggaaa atcatcatca ttaacatcag taccatctaa attaagagct tctaattgtt 360

ttaagggaag gtggaagcta aaatgaaccg gcccatccaa cagagggcac tatctgtgga 420

ggaccaacaa aggaaaacag agcggaaaat atgggcgcac atgtgcctcc agttcatgaa 480

gttatttgga gggagggggg aagaattaaa cttacattat tgattacaaa aacagttgaa 540

aatctgttag agtgaaaaca gccatggtaa tctacagaag aaaagggaga atggaaagta 600

aagatcattc atgtcttgaa ataagttgtc tacagtctgt gagaacttgt gggaggtgat 660

cctagtgtga tatattagtg catgagccta gcaaggaaat cacatcacca cagggcttag 720

cgcttagaat tagaactaag tcctcattat ttgtgagaaa ctcaaaacag aacttaacaa 780

aagtgtttaa caactttcaa tttttaagaa gggacgctat ctcattttca gacgccatgg 840

atcctcacta gcaaaacaca atgtcacaga caaacgaggc atatcaggtt tttctaactc 900

atttcggtac tccatcactt aatcatgaaa taatgttgac tatatatata tatatatata 960

tatatatata gtcaacatta tgggtttttt ttgttttttt tttttttttt tttttgagac 1020

ggagtctcgc tctgtcgcct agactggagt gcagtggcga aatctcggct caccgcaacc 1080

tccgcctccc gggttcaagc gattctcctg gctcagcctc ccgagtagct gggactacaa 1140

gcgcccgcca ccacgcccag ctaatttttg tatttttagt agagacgggg ttttactatg 1200

ttggccaggc tggtctcgaa tccctgacct cgtgatccgc ccgcctcagt ctcccaaagt 1260

gataggatta caggtgtgag ccaccacacc cggctctgat gtttacaata ttataccaac 1320

aagggttgga tcgtgtgtga cttatctaga ggttaagagc aaaagttatg aaatcacagt 1380

gccatcatgg gagtctagct ttcaccattt aaagttgtgt aggcagccag gcgtggtggc 1440

tcacgcctgt aatcctagca ctttgggagg ccaaggtggg cggatcacct gaggtcagga 1500

gttcgggacc agcctggtca acatggtgaa accccatctc tactaaaaat acaaaaatta 1560

gctggggttg gtggtgtgcg cctgtaatcc caggtactcg ggaggctgag gcaggagaat 1620

cgcttgaagc tgggaggtgg aggttgcagt gagccgagac catgccattg cactgcagcc 1680

tgggcgacaa gagtgaaact ccatctcaaa aataaaaaaa aataaaaaat aaagttgtgc 1740

aggccgggcg cggtggctca cgcctgtaat ctcagcactt tgggaggccg aggtgggcag 1800

atcatgaggt caggaatttg agaccagcct gaccaatatg gtgaaactcc gtctctacta 1860

aaaaatacaa aaattagcct ggtgtggtgg cgcattcctg taattccagc aactcaggag 1920

gctaaggcag gagaatcgct taaatccggg aggcggaggt tgcagtgagc caagattgtg 1980

ccactgcact ccagcctggg caacagagca agactctgcc tcaaaaaaaa aaaaaaaaaa 2040

aatatatata tatatatata tagatataga tatagatata tgtgtatata tacacatacg 2100

tgtatatata cacgtatata aagtgtgaac ttgggaaagt tatttaatct tattgtgtgt 2160

tagtttcccc atttataaca tgggtgagta atatgtatcc tatttcttag agtcaatggg 2220

aaggttaaat agcaaaatga aggtaaaagt aattagaatg taacaggcac attacaatca 2280

ctcaataaat attcttctgc ctcagcctcc cgagtagctg ggactacagg tgcgcaccac 2340

catgcctggc taatttttgt atttttagta gagacggggt ttcaccatat tggctaggct 2400

ggtcttgaac tcctgacctc gtgatctgcc tgcctcggcc ttccagagtg ctgggattac 2460

aggcatgagc caccgtgccc agccggctga ctattttctt aagaataagg atagttggtc 2520

acctcgggag gctgaggcag aagaatcgct tgaacttggg aggcggatgt tgcagtgacc 2580

cgagatcccg ccactgcact ccagcctggg tgacagagcg agatgcggtc tcaaaaaaaa 2640

aaaaaaaaac aaaaaacgaa aaaaaagaaa aatagtcggc cggggacggt ggctcacgcc 2700

tataatccca gcactttgag aggccgaggc gggggcggat cacttgcggt caggagttgg 2760

agaccagcct ggccaacatg gtgaaacccc gtctctccta aaaatacaaa aattagctgg 2820

gcattgtggc gcaggcctgt aattttagtt actctggaag ctgagccagg agaatcgctt 2880

gaatccggga ggtcgaggtt gcagtgagcc gcgatgaagc cattgctctc caacctgggt 2940

gacaagagct agactctgtc tcaggaaaaa aaaaaaaaga aaataggaaa gctgcccgca 3000

aagcctgcac cttggacacc tcgtctgcac taacatttgg gaaattcttc ttattatttc 3060

tccggaacat atatatttac attctttaca tgtctgtttc acgttaaaga aaatatttct 3120

ggagcaatca attttttttt cctttttttt tttttttttc tgagacggag tttcgctctt 3180

attgcccagg cggagtgcaa tggtgcgatc tcggcccact gcaacctccg cctcccgggt 3240

tcaagcgatt ctcctgtctc agcctcccag atagctggga ttagaggcat gcaccaccac 3300

gcccggccaa tttttttttt tttgtagtag agacggggtt tcgccatttt gcccaggctg 3360

gtatcgaact tctgacctca ggtcatctac ctgcttcggc ctcccaaagt gttgggatta 3420

taggcgtgag ccactgcccc tggcttccaa tttttttttc cttttttttc ttttcttttt 3480

tttttttttc tgagacggat tttcgctcct gttgcccagg ctggagtgca atggtgtgat 3540

ctcggcccac tgcaaccttt gcttcccggg ttctggcgat tctcctgcct cagcctcccg 3600

agtagctggg attacaggca tgagccacca cgccccgcta attttgtatt ttgtatttat 3660

ttatttattt attttcgtag atacgtggtt tcgccatgtt ggccaggctg gtatcgaact 3720

cctgacctca ggtgatccgc ccgccttgcc ctcccaaagt gctgggatta caggcgtggc 3780

catcgcgccc ggccaaatat ttttcaaaat ctaattttgc ataatgacca agatttgcag 3840

aaaaatacaa attaaagaga tcttttaaag taaagattcg tcataaaagt cacagaaata 3900

atactcatga ataataaatt agaatgggca atgttaagta cttgaaataa caaaggtttt 3960

agagtcaggg tttctgcagt ttccctagcc atataacgag gagactgtta gagaggatat 4020

cctaaaatgc tttccaagtt tacccacctc taactcggaa gaaggcaatt taaactaatt 4080

tggtttaccg gaattcaaat acgtacaatt caacataatg aattgaactg aaattgaaca 4140

cctgcttgcg taaacctagt cggctcgagt tgttctggtt ctggattggt cagtctagag 4200

ttccaggggt gtggccaaaa gtctgtgagg cggggccgta ggcagcctaa atcaggcatc 4260

ggcgcggtca gcctcgtggc gcgcccacgc ccccacgccg gctcttcccg gggtccttcc 4320

gtgcgcgttg atatgattgg ccggcgaatc gtggttc 4357

<210> 20
<211> 9648
<212> DNA
<213> Homo sapiens

<400> 20
aaaggaaagc taggggccac cctggccgct tttcagggtc tgcgcaggcg ccttgagcac 60

tgcgtcgcga cgggagagag ctttacggct tctgttgcgg ctaacgggcg gggcgtctgg 120

tcagcggcgg gaaggccctt aggcgctcag agcgggcggg ccctgtgagg cggaagtggc 180

gggctctgtg ggcggaaggg gcggggcctg ggagtgtcgt gtgcggactg aagggaaggc 240

tgcgggagtg tgcttcgcgt cctctgtagt cgactaccgc agctcttctt tgcattctgg 300

ctctttaata atctcccttc gccctttctt taaatcagtt cccaccctcc caacacctgt 360

aatctttctc ccctctggac tccctcctcc ccgaatcccc cgcatcagaa tcttcctttg 420

gaatctactg caagaaatct ggctggattc tcagattaag gacttccaga atcctatcct 480

ccctagaatc cccctccttg gggatcctct cccagaatcc ttttcccctc agagcccctt 540

cccctcagaa tctccctttc accagaatgt ccttcctttc agtttcctct tcccagaatg 600

tttcccatcc ttctctcaga tggccaaccc caaatgtccc tcttaagagt gctcaactta 660

gacccccctt ttccctgaat ctccctccat tcggtcctcg tcccctcagt cacccttccc 720

agaatctttc tcttgtcagg acccagtacc cctggaatct ccctcctcca gaatctccct 780

ttcccggcaa atcccccttt cccggcaaat ccccctttcc cagaacaccc cgccccgcaa 840

atccacaaaa acttttctct cagagacccg tccctcaaaa tctacctttc tcctgagttt 900

atttcccttc cgagactcct cttcccagca actgccttcc ctggagtttt ccctccgtta 960

gaatccccct ctcctaagag accctccccc aagaattaac ctcttccccg tccccaccct 1020

agaatctctc tctttttttt gagacggagt ttcgctcgtt gccctggcta gagtgcagtg 1080

gtgcgatctc gtctcactgc aacctccgcc tcccgggttc aagagattct cctgccttag 1140

gctcccgagt agctggaatt acaggcgtct accaccacgc cctggtcgtt ttttgtattt 1200

ttagtagaaa tggggtttca ccacgttggc caggctggtc tcgaactcct gacctcaggt 1260

gatgtgatcc gcctcagcct cccagagtcc tgggattaca ggcgtgagcc accacgcccg 1320

gccgaatctc tctgtttttt gttttttttt tttctttttt ttgagacaga gtcctgctct 1380

gtcgcccagg ctggagtgca gtggcatgat ctctgctcac tgcaacctct gcctccccgg 1440

ttaaagcgat tctcctgcct cagcctcttg agtagctgag attacaggca tgcaccacca 1500

cgcccggcta atttttgtat ttgtagtaga gacggggttt caccatattg gccagactgg 1560

tctcgaactc ctgacatcaa atgatccact catctcagcc tcccaaagtg ctgggattac 1620
aggcgtgagc caccgcgccc agcccctaga atctctttct ccagaatctc ccagtcatct 1680
ttttcctctc ccccaaatca ccctccactt aagcctcctc cccacagaat tctccatccc 1740
cagcccccca accccacctg aggaacccca ggattgggtt gtcctctggt tccccggcag 1800
atgtccctgc gtctcacccc atcaagagtc tccccagcga agaggttcct ctctcagagc 1860
tagagggagc ccacagttgc ctcagtttct ctggcagtac agggtctggg atccagggtt 1920
caaaagggcg tcgtggtgca ccttgccaaa gagggtggag acaggagacc actggagggc 1980
aggtctgggg ccagagggaa aagccagccc ggagtccagc tctaaaaggg tgagggggga 2040
gggggctgtt gggagggagg aaggaagtac ccagaggctg accgggaggg acacagacac 2100
agcccaaaga aatgtaggca caagtggaga agaagagaaa gatgaaaggc caagggagaa 2160
gaggaggaga aaagggagag accaggacag tcaagagatg gacccccaaa acaagcccag 2220
tgacagattt agaggcagag aggccaaccc tcagacaaaa aagagataaa ggcaggcatg 2280
atgtttccaa aatacttgag acagaggcca ggtgcagtgg ctcacaccta taatcccagc 2340
actttgggag gccaaggcag caggatcact cgagcccagg gttcaagacc agcctggcca 2400
acatggtgaa accccgcctt tactaaaaat acaaaaatca gctgggcatg gtggcaggtg 2460
cctgtatccc agccacaagg gaggctgagg caggagaacc gcttgaaccc gggaggcgga 2520
ggttgcagtg agctgagatg tcgccactgc accctaggct gggcgccaga gcaagactct 2580
gtctcaaaaa taaataaata aataaactct gtctcaaaaa taaataaata aaataaaata 2640
aatagagcag agacctcaaa tattcacttg cagacacaga taaaggctag cacaggtgtt 2700
ttccaatgaa gacacaggca gatgagagga tgcacacatt ctacctgtac tttttctttt 2760
ttttttttta aattgagata gagtctgtgt cacccaggct ggaatgcagt gatgcaatct 2820
gagctcactg caacctccac ctccccggtt caagtgattg tcctgcttca gactctggag 2880
tagctgagat tacagattct cctgcttcag actcctgagt agcccagatc acgggcctcc 2940
accactatgc ttgactaata atttttcttt tttttttttg agatggagta tcgctctgtc 3000
gcccagactg gagggcagtg gcttgatctc agctcactgc aacgtcagct tcctgggttc 3060
aagcaattct cctgcatcag ccttccaagt agctgggatt aaaggcatgc accaccatgc 3120
ccagctaatt tttgtattta ttagtagaga cagggtttca ctatgctggg cagactagtc 3180
ttgaactcct gacctcaagt gatccacttg cctcggcctc ccaaagtgct gggattacaa 3240
gtgtgagcca ctgtgcctgg ccactaattt ttgtattttt tagtagagac agggttccat 3300
cgtgttggcc aggctggtct tgaactcctt acctcaagtg atccgcccac ctcggcctcc 3360
caaggttctg ggattacagg tgtgagccac agcggttggc ctagcctgca ctgtcttaag 3420
ggcctgttgg gcaggggagt tcaggttctc tcgtctctcc agattgatct tcacacattt 3480

gctaagtctc gtatcgtccc tgagcttggg agtcagggag cgctctgacc taagccctcc 3540

tgactttggg gtcaccatct ttccccagcc tctatgggca cactccctga attatctgtg 3600

gcttgagtgg agcaagcagg ggatgggatg aggggtggcg ggggagttcc cctcatccac 3660

ttcagccttc cagtctgctc ccccttcctg tacctctctc ttccagaacc ccaggccaag 3720

caccagagaa ggcctgggag tgagcaaggt gcccatgttg cttctgtgga tcatcctcct 3780

gcttgtacag ccctgggagg gcctaggaac gcccgcccct ctgctggagg gagccttttt 3840

acttccttgc agccctctga agataccagc gagtggggag cagagccagc agacggggtg 3900

ggccaattga tggggcgtgg tagggggtgt gtttgaggaa gggtcagcct ctttccctcc 3960

aagatctgat tctcctcctt acccctgaac tgtaggaaaa caaaaatgat gacaccctct 4020

acaccccaga tgatctctca gtgagtattg cattcgcttc tttctgcagt tgcaggtttt 4080

gagagaattc agtggtctcc atgtgtgagg gctgctgtgt gaccatatga atatgttgcg 4140

gggaggaagt agtgggacta tctcctgacc cagcccctag ccttgacaga tatattttgc 4200

tcactgctgt ctccaacacc tagaataggg aggtatataa taacaaatga tgatgatgac 4260

tacagctgca tgtaaatagt gtttactctg tgccaggtat tggtttaaat gctttaagta 4320

tgttagctta tttactctgt acaccaatcc tataagatag ataggaccac catgtatgga 4380

tgtgaatgtt gtacactgca caaggatccc acatctacaa agacagtact atattcacag 4440

gctggatatt gtagatttgc atatttgcca acttgcggca gatggcagta aagtatcttg 4500

ttctaataaa atcaagactt agaaggtaga aggaacttaa ggaaggtctt tttattttct 4560

tttttttgag acagggtctt gctctgtccc ctacgctgga gtgcagtggc acaatcacag 4620

ctcactgcag tcttgacctt ttaggctcaa gcaatcctcc cacctcagcc tccagagtag 4680

ctgggaccac atacacgtga caccacaaat agctaatggc taattttttt tttttttttt 4740

tgagacacag tgttgctctg ttgccagctg gagtgcagtg gcgtgatctc tgctcactgc 4800

aacctctgcg tcctgggttg aagcaattct tctgcctcag cctcccgagt agctgggact 4860

acaggcatgt gccaccatgc ccagctaatt tttgtatttt tagtagagac agggtttcac 4920

catgttggcc aggatgttct cgatctcttg acctcgtgat ccacctgcct caacctccca 4980

aagtgctggg attacaggcg tgagccaccg tgcccgacct tttttttttt tttttttttt 5040

ttgtagatat ggggtgtcgc tgtgttgccc aggctggttt tggacacctg ggctcaagtg 5100

atcttcctgc cttggcctct caaagagctg ggattacagg catgagccac tgtgcccagc 5160

tgagtgtctt tttctagctt gcacataagt acagcatggg atagtgtcag gccttacggt 5220

tgggattatc cagagcccta tttacagatg aggaaactga ggcccagaga actgaagggg 5280

tttgttccag gacacagata agaaatcttg ggagtgggga cttggactca tgcccttacc 5340

catagatacc aggtcacttt gtccctactg tgtgtcctgc tgagactcta ggctgctcca 5400

ggttggagct gtctgtgatt gggtttgagg agggaccatc tatgggaact gctgtgttcc 5460

agctacagta cctgctggat ctcctggggt cccggctgtg ggtggccagc cagggccctt 5520

gcctgtcctg tgaaggacat ccccagagac ctgtccccct cttggagtta ctacagggag 5580

cttgtgcttg gcaccctacc acagcctgag ctttggagga catgggctcc caggtacacc 5640

cagagaggct gggtagagag agggagggaa tagtcttcag ctccaactgc caaaccctaa 5700

gccttttctt ccctctaggc cagaggaccc cacctggagc cctagtagcc aaggggaaga 5760

gttaagaagg aggaaagtaa ggtgcgatct gaagtctctg acctcttggg cagggagttg 5820

gataaattgc cccacctgcc cccattctca ccctccttta gatactcttc tctcttcttc 5880

caggaaggga cagagaagtg cacaggtatt ctctaacctc atccctatac cttgagaaca 5940

gggagggtcc tgtggttcac atccctacat ccctggctac aaaaaattac cacttgaaga 6000

actttctttt cttttctttt ctgtttttga ggcagagtct cactctgttg cccaggctag 6060

agtgcagtgg catggtctca gctcgctgca acctccacct cctgggttca agtaattctc 6120

ctgcctcagc ctcctgagta actgggatta caggcacgcg ccactacacc tgactaattt 6180

ttgtattttt agtagagatg gggtttcaca atgttggtca cgctggtctc gaacgcctga 6240

ccttgtgatc tgcccacctc agcctcccaa actgctggga ttacaggcgt gagccaccgt 6300

gcccagccca ccacttggag aactttcaag taatactgat ggccagaccc cagcccagat 6360

aatttgataa ttggtctctg gtgaagcctg agcatctata ttatttttat tattatttag 6420

agacagggtt tcactatgct gcacaggctg taacttggca tctgcccata tatttttttg 6480

agacagggtc tcactctgtc acccaggctg gagtgcagtg gcatgatcaa aactcattgc 6540

aggctcgatc tttcaggctc aagtgactct cccacctcag tcccccaagt agctagcgct 6600

ataggcgcat gccaccacac tcagctaatt tttgtatttt ttgtagagat ggggttaccc 6660

tttattgccc aggctaatct tgaactcctg gctcaagcga tactcccccc ttggcctccc 6720

aaagtgctgg gattataggc atgagccacc acacctgaca tttttttttt ttttttgaga 6780

cagagtctcc ctttgtcacc caggctggag tgcaccggct ggtggactgg atggccacat 6840

ttccatcacc ccaaagcatt ccttctctct cttcccagtc aatttccacc tccctctcca 6900

gccccagaca tccactaatc tgcattgtct gtataggttg gttttgcttg ctctggagtt 6960

tcatatgaat ggaatcatgc tggacatctc ctgatgtctg gcttctttca ctcagcataa 7020

tgttttggag attcagtcat gggtgtattg gtagttgtta ctattctgtt gcatggatgg 7080

atgtaccaaa atttgttttt cttttgagac gagtctcact ctgttgccca ggctggagtg 7140

cagtggtgcg atctctgctc actgcaacct tcacctccct ggttcaagtg attctcctgc 7200

ctcagcctcc caagtagctg ggattacagg cgtgcgccac cacacctggc tatttttttt 7260

tttttttttt ttgtattttt agtagagatg gggtttcacc atattggcca ggctggtctt 7320

gaactcctga cttcgtgatc cgcctgcctt ggccttccaa agtgctggga ttacaggcgt 7380

gagtcactgc gcccggccct aatttttgtg tttccagttt agggctatta tgcaactctt 7440

tctttctaaa atattctcaa gtattctgat gtgctaccag agttgagttc agaaccacgg 7500

atccaccaag gaagaaactt ccatggctgg ggaaatgact gtgccctctt ttttctgcag 7560

ggaatggctt tctgcagcag tgtctgtagc gatttaatat gtagaatcct attttcttgt 7620

tgtttttttt tttgagacgg agtcttgctc ttttgtcaca gcaacctcca cctctcaagt 7680

tcaagagatt ctcctgccac agcctcccga gtagctggga ttacgggtgt gcgccactac 7740

gccacctggc taatttttgt attttttagt agagacaggg ttttaccatg ttggccaggc 7800

tggtcttgaa ctcctgacct caggtgatcc gcctgccttg gcctcccaaa gtgctgggat 7860

tacaggcgtg agccaccgcg cccggtggag aatcctttct ttttttgaga tggagtctag 7920

ctctgttgcc caggctggag tgcagtagca cgatctcggc tcactgcaag ctcggcctcc 7980

cgggttcacg ccattctcct gcctcagcct tacgtgtagc tgggactaca ggcgcccgcc 8040

accacgccca gctaattttt ttttgtattt ttagtagaga tggggtttca ccgtgttagc 8100

caggatggtc tcggtctcct gacctcgtga tccacccgcc tcggcctccc aaagtgctgg 8160

gattacagac gtgagccacc gcgcccggcc caggtgtgtc atacttatgt gagaaatttt 8220

accaatgtgt cctgaggctt cttttttttt tttttttttt tgagactgag tctcactctc 8280

gcccaggctg gagtgcagtg gcgcgatctc ggctcactgc aagctccgcc tcctgggttc 8340

acgccattct cctgcctcag cctcccgagt agctagaatc acaggcacat gccaccacgc 8400

ccggctaatt tttttgtatt tttagtagag acggggtttc accatgttgg ccagactggt 8460

ttcgaactcc tgacctcagg caatctgccc gccccggcct cccaatgtgt tgggattaca 8520

ggcatgagcc accgcgactg acatagactt ctgattttta aaatttcccc tatcaggtta 8580

atttttcaat ttttaaaaat ttaagtttta attttttttt tttttttttt ttagagacag 8640

ggtctctatt acccagtctg gagtgcagtg gtgcaatcat agctcactga agcctcaaca 8700

tgggttcaag tgatcctccc accttagcct ttggagtagc agggaccaca ggcacacgcc 8760

accacaccac attaaaaaaa acacaaaaat tggatacatg gggtctcaat ttgttgccca 8820

tgctagtctc aaatacctgg cctcaggtga tcctcccgcc tcagcctccc aaagcgctgg 8880

gattacaggc gaaggttcat ttttcatctc tccattacac ctgtccactc tgccatcact 8940

cgtcagccgt cattactaat agataaattc gcctttaaat gaacttagga agcctggaaa 9000

gaaaaatagg gacttgggcc gggcgcggca ggttatgcct gtgatctcag cactttggca 9060

ggacgaggcc ggaggatggc tttttagttt gagttagagg agtttgagac caggccgggc 9120

aacagcccga ctctaccaaa aaaaaaaaaa aagtacaaaa agaaagaaaa agaaggaaaa 9180

accaggcttt cacggtcact cagacttggc gaggaggctt gggtctgccg ctttccactg 9240

agtttttctg agcttcagcg ttctctccaa aacggggata cggatctcct ggggatgctt 9300

tagagggcct aggagataaa gccttgtttc ttttataatt tgctattcgc tgatttcgcc 9360

gcctcctcgc agcttggcct accgcgctag tgttccggac taacgaacaa acggctcact 9420

aaattcaaac atgagtttca gcctggtcgc cgagccggcg ggtagaaaaa acctttcccc 9480

tcctaaccct ccgcagatgt cggccccgca ctccgcccag gccagccctg cctctctcaa 9540

gatggcgacc tggaagtgcg tcgcccgtgc cagccccgga aatctcgtgt taggcgaggt 9600

caacgggtag gttctcgcga gaggacccgt cagccccagt caggcgtc 9648

<210> 21
<211> 12561
<212> DNA
<213> Homo sapiens

<400> 21
   gaagaagctg gcccacgcat gcgctcttca aatttttgag acagtttacc cagaatgcag 60
   tgctcaaagg aaacgcgtgc gcagtgtggt caggttgttt cgctgggtga gtaaaatgaa 120
   atcttagagg cgttgtgggc tggcccagtt gatgacgtca ccataccaca gcttctagtg 180
   ctattctgcg ccggtatccg accgccgggc gcggtggcgc gtgcctgtag tcccagctac 240
   tcgggaggct gaggctggag gatcgcttga gtccaggagt tctgggctgt agtgcgctat 300
   gccgatcggg tgtccgcact aagttcggca tcaatatggt gacctcccgg gagcggggga 360
   ccaccaggtt gcctaaggag gggtgaaccg gcccaggtcg gaaacggagc aggtcaaaac 420
   tcccgtgctg atcagtagtg ggatcgcgcc tgtgaatagc cactgcactc cagcctgggc 480
   aacatagcga gaccccgtct cttttgaaca ataaatacgt taattttgga ctctcaagag 540
   atgaatattc tcatagtctt ttttattgat taaacctttt actttctttt aattaaaacg 600
   ttacacttgg gaaaggagtt tttcctcgac tcaccagtga cacctactgg aataaattta 660
   caaaacaact taaaatcagc tgtaatgatc acgagtcaac ggtagggttt gcttttattt 720
   tgttaaagag agtacgtttt caaatttatt tttttgacac actcctccaa gaaatacacc 780
   tacaccattc caaaaaaaaa tgagattctt tggcatttag tgtatttgat tcttaaatgt 840
   aattggctga atgtaaagta ataaacaagt aatgtgcagt gtatgttgct ttgaccagat 900
   aatttggtga caaaacaagt aatgaaaagc aaatgcaaaa atgcaaaaaa ccaatgtcaa 960
   taaacgaatt tctgctgaaa gatacctaca ttccatatgg tcatttataa tgcctccaga 1020
   taaaactgct ctgaaatcaa agctttaact agatttataa aatgtagcac agggtgcaac 1080
   aattccagaa acttttacat ataaactttt ataaattgaa atacaacact tctttaaggt 1140
   atgttatgtc agggttctag tggggtcccc ctacccaatc attaagttag gcattgtatt 1200
   atatgattaa ggaaggaggg gagtcagaga tttgtaaact agatataact tgctccttgc 1260
   cctcaaaatt ttctaatctg tgggaaacag gattatctcc aagacttcaa catacaacgt 1320
   ttgtgcacca acaacatatg tacagctaag gaaaatattc tgtattaaat catgggtgtg 1380
   ctcatgaagt acgaaaggag ccaagaagag aaattaattc cagttcgagg tacatttgag 1440
   agacctttga ggtagacctt gaagaatggg atttctacag gcagcacttg acagaaggga 1500
   atgcctggtg aaaggaagcg cttgagccaa ggtaaggtga acaactttgt gtaactagaa 1560
   tagaagatgg acaaaaggcc tagtgatagg aatgaaaagg tattttattt taaagcctca 1620
   ttgggcaaga tccaatgtca agcttttaca actttcctat aatttatatc agtcactaat 1680
   gtgtggtttc caaaagccat ttgcctctca aaagctaatc taacatttgt ccacactttc 1740
   ttaggcctct tctagttggt ctccacaata tttaaaacat tacagtggtt ctgagatcaa 1800
   ttccattaat atttttagta ctctgatata taagcctttg ggcctaaaga agtgaattca 1860
   tttaggagag aaaaaaagac aaatgagaag caattagttg cacttcaaca atcttaatct 1920
   gaatacttaa gtaattctta atacttattc tagtctatac attattctct ttgatagaga 1980
   tattgcaaaa gtgcagctaa gacgtcgtac ttcctttcag ttttaagata atgtgacacc 2040
   atctatccca aaccatgaaa ttatccattt ctttttcaac aaactgagaa cacagcttta 2100
   aaagttcatt ttaactcggt gtttatcaag agcttttatg ttttgttttg ttttttttcc 2160
   tttttttttg agacggagtc tcactctgtc gcccaggctg gagtgcaatg gcaccatctt 2220
   ggctaactgc aacctgcacc tcctgggttc aaggattctc cctgcctcag cctcccgagt 2280
   tgctgaaatt acaggcacgt gccaccacac ccgtctaatt ttttacattt ttggtagaga 2340
   tggggtttca ccatgttggc taggctggtc tcgaagtcct gacctcgagt aatccaccca 2400
   cctcagcctc ccaaactgct gggattacag gagtgagcca ccacgcccgg cctaagagct 2460
   tttatcttaa tcctgtagtc ttcttacagt ttctgagtcc tttttttttt tttttttttt 2520
   ttttttgaga cggagtctgg agtctcgctc tgtgacccag gctggagtgt agtggtgcga 2580
   tctcggccca ctgcaagctc cgcctcccgg gttcacgcca ttctcctgcc tcagcctccc 2640
   gagtagctgg gactacagcg cccgccacca tgcccggcta atttcttttt gtatttttag 2700
   tagagacagg gtttcaccct gttagccagg atggtctcga tctcctgacc tcatgatccg 2760
   cccgcctcga cctcccaaag tgctgggatt acaggcgtga gccatcttag ttctctttta 2820
   tacatgggtt tgaaatgctt cttcttttag aacacatttt cctttaatag gatatttatt 2880
   gggtttgggt tctttgtttt ttaaaaaatg tacaatgaag tgcaaagaaa aagaaaaaaa 2940
   aaagtgccat aatgtccttg cccagataac cctactaata tttttctttt gaaaaactaa 3000
   aagtaatata catgtaagat cgaagcattc taacactgca aagtatagat cttattttta 3060
   agtacattgg aataatatgc atgttaggac actattattc aagatgtctt taattttctt 3120
   tctcaaatta ttcttacagt cttctgtgat catccatcca gaatttaatc atttaaaaac 3180
   cttcatctta gacgtataca ctgctgatgg gaatgtaaat tacttcagca ctgtggaaag 3240
   cagtttggtg atttcttaaa gaatttaaaa cagaactgtc atttgaccag caatcccatt 3300
   actggatata tacccaaaag aatataaatc attctacagt aaaggcaaat gcatatgtat 3360
   gttcatcgca gcactattca caatagcaaa cacactgaat caacctaaat agcaattttt 3420
   taagcaacag tagactggat aaagaaaatg tagtacatat acactatgga atgctacaca 3480
   gccacagaaa agaacaagat catgtcctgt gcaacagcat ggatggagct ggaggccatt 3540
   atcctaaaca aactaacaca ggaacagaaa accaaacacc acgttctcac ttataagtga 3600
   gagctaaaca ttgaatacac atggacccaa agaaagtaat agacagcagg gcctacttga 3660
   gggtagaggg tgggaggagg gtgaggattc gaaaagtacc tattaggtac taagcttatt 3720
   acctgggtga tgaaataata acccctgtga cacacaattt atctatagaa ccaacctgta 3780
   catgtttcct tgaaactaaa agttttttta aaaacacctg gccaggcatg gtggctcacg 3840
   cctgtaatcc cagcactttg ggaggctgag gcaggcagat cacaaggtca ggagttctag 3900
   accaacctgg ccaatatggt gaaacccggt ctccactaaa aatacaaaaa ttagccaggc 3960
   atggtggcgg gcgcctgtag tcccagctac tctggaggct gaggcaggag aatcgcttaa 4020
   acccaggagg cagagcttgc agtgagccga gattgcgcca ctgcactctg gcctgggcaa 4080
   cagagtgaga ttccgtctca aaaaaaaaca aaaaacaaaa aaacctgcat cgctcttgag 4140
   ttactttcaa ttttggaacc tttgggaata gaatcatact tttcctctaa actttacaaa 4200
   acctatttta ccatagacag agtctaaaat acatgtttcg catgtttggt tattacaata 4260
   tgcacacttc agtatcatgc cccggttttt tccagttggt tagaattagg tctttttttt 4320
   tttttctttt ttgagacgga gtctcactct gtcacccagg ctggagtgca gtagggccat 4380
   ctcggctcac tgcaacctcc acctcccagg cttaagcgat tctcctgcct cagtctcctg 4440
   ggtagctgag atcacaggcg tgtgccacca ctcccagcta atttttgtgt ttttagtaaa 4500
   gacggggttt tgccatgttg gccaggctgg tctcgaactt ctggcctcta gtgatcttcc 4560
   ccctcagcct cccaaagtgc tgggattaca ggcatcagcc accacgccca accagaatta 4620
   ggtctttatt aataattatt aacccttcaa aaaagcccag ttgttcaata gtgttatgtg 4680
   tttgtccata tcgattttag caaatattaa tattttaatt aactgtacat attatatttt 4740
   aaagtaaaac atacatactt caggaaaggg gactcagaaa aaggaaaaaa atacatacat 4800
   acatgcatac attaaatata ttatcaaaag taagtctttc tccaacccta gacccctatt 4860
   ttcccagtct ccctgtccaa accaacaagt tagcagtttt tagaggtttt ctgtttgttt 4920
   gtttgtttgt ttgtttgttt gttttcacag agtctcactc tgttgcccag gctggagggc 4980
   aatggtgcag tgcaatctca atctcagctc agggcaacct ccacctcccg gctcaagcga 5040
   ttctcctgcc tcagcctccc aagtagctgg gattacaggc gcatgtcacc acacccagct 5100
   aatttttgta tttttagtag acacggagtt tcaccatgtt ggccaggctg gtcttgaact 5160
   cctgacctca ggtaatccac ccgcctcagc ctcccaaagt gctgggatta caggcatgag 5220
   ccaccatgcc tggcccaatt tgattttttt tttttttttt ttgagatgga gtcttgctct 5280
   gtcagccagg atggagtgca gtggcgtgat ctcggctcac tgcaacctct gcttcccggg 5340
   ttcaaacgat tcacctacct cagcctcccg agtagctggg attacaggcg cccaccacca 5400
   tggctggcta attttttgtg tttttagtag agacagggtt tcaccgtgtt agccaggatg 5460
   gtctcgatat cctgacctca tgatccgcct gcctcggcct cccaaagtgc tgggattgca 5520
   ggcatgagcc accacgcccg gccccaattt gattttttaa agtccactta gcaagattca 5580
   atttgatttt ctgatgagac agagcgtatt ttcctatgtt tataagccag acatatatct 5640
   ttatctggga acttttttga gtccacagcc caattttttt aatccattgg taagctagtt 5700
   tatatattta taaatactta tgtataagga aatacacctt tgaccttcat ataagatgca 5760
   aagttatttt ctttgccacc atcccagatt cttacttatt ttttaaattt gtttatggta 5820
   tatttttcca tgcaaaaagt tttttttttt atttttattt tttggctagt caagtgaagc 5880
   agtgggagta gagaaggaac aaataaatct gtaactggtt gtaattaatt agttgtaaac 5940
   tccactgcac tcggaccagc accatgcaaa aagttttaaa gtctatgttt tttttttttt 6000
   tttttttgga gacggagttt cactcttgtt gcccaggctg aagtgcagtg gcgtgatctt 6060
   gcctcacctc aacctctgtc tcccgggttc aagcgattct cctgcctcag cctcccgagt 6120
   agctgggatt acaggcatgc gccaccaccc ccagctaatt ttgtattttt agtagagacg 6180
   gggtttctcc atgttggtca ggctggtctt gaacccccgc cggccaaaag tctatgtatt 6240
   aaaattaact tgccgggcgc agtggctcac acctgtaatc ccagcacttt gggaggccga 6300
   gacgcgtggg tcacctgagg tcaggagttc gagaccagcc tgaccaacat ggagaaacca 6360
   cgtgtctacg aaaaacacaa aattagcccg gcgtggtggt gcatgcctgt aatcccagct 6420
   actcaggagg ctgagacagg agaatcgttt gaacccagaa ggcggaggtt gcagtgagcc 6480
   gagattgcgc cactgcactc cagcctgggc agcaagagcg aaaccgcatc tcaaaaaaaa 6540
   aaaaattaaa attaacttgt cacactactg cctttaggtt ttgcatcaaa attctttaaa 6600
   ggttacatgt gttttcctgt gatattttta aagtttttat ttaaatcttg aaaccaccta 6660
   aaatttatgt cactgttccc caggaatgaa ggagaaattc agcttttttc tcctcccaaa 6720
   tggctactta tcccaacaca agccgtatta attgcatgta aggctgctgt attctagcac 6780
   aatgcatgct ttcagaaata tttgctaaat gaataaatga aaagcagtat ttgtttattc 6840
   tttctacaaa tatgcattga gtaccgactg catataacca ctatgctagg ctttgcatat 6900
   aacagtaatg actcagaagc caggaagcat atcatctagc ggtgaagata agcattaaat 6960
   aagtaatcat atagcaatat tttgaacagg ataaaattcc tcttacagtc aacactcaaa 7020
   tgtgctggat aaaatatgac attttggccg ggcgcagtgg ctcatgcctg taatcccagc 7080
   actttgggag gccaaggtag gtggatcacc tgaggttagg agtttgagac cagcctgact 7140
   aacatggtga aactccgtct ctactaaaaa tgcaaaagtt agccaggtgt agtgtaatcc 7200
   cagctactca ggaggctgag gcaggagaat cgcttgaacc tgggaagtgg aagttgcaat 7260
   gagccgagat cacggcatcg cactccagcc tgggcgacag agcaagactc tgtctcaaaa 7320
   aaaaaaaaaa aaaaaaaaaa gacgttttaa aatgcagagg tgagctcaca agaaaggggt 7380
   atactggggg caggtgctaa gtgggaactg aaaaaacaga ggctgtcctg gggcaatctg 7440
   tcctcctaag taatattaca tataacctgg attccaaagg gagcaagaga caagcctttg 7500
   ggcaactgta tttaatcaaa ttgtgatgag tagaaatcca tgaaggtttt tgggagaaat 7560
   taaatacagc atctgagact tgagttctag tcctgtttat tctaacattg actgtctatg 7620
   aggtcatggt caaatagtgt acctttactc ttaattccat tccctcttgt gcaaaatgag 7680
   agaattgaag taaaatatct ataaacgaca tttcagctct aaaacttgag gattctatga 7740
   taggatatcc ctctacaaat ttaatttggc tgcagtatgg atggtggatt attacaacag 7800
   cctcctaact gatacatctg ctttcaggct tgctctacct ccaaaatcat ctttttactg 7860
   accacaagaa cctttcctga tcaggtctga attttatccc gcttaaaatt tttcaaaggt 7920
   tttccagaac tttcagagcc tgggggacag ggtgtgaacc tgtctcaaaa aaaaaaaaaa 7980
   tcctaagtgt taaatgaatg aataaatgta ttgaagcaag aagacctggt aaatttagca 8040
   acagctctgt agcaaagtaa tgcagctctg aattgatacg gcagtgctaa gaatggaaag 8100
   gtggtcattc attagtaaat aatcgtggaa acacaatcta taagacattc aaagagtgtc 8160
   actgatgaaa ggcagaactt agaggagtga aagttgcaga taccaggatg aaatcacttt 8220
   tgtcagaacc aaacaaaata gggccagaaa ggcaaaaagg aaagggctca tgtttgtatg 8280
   tctgagataa gaactgtttc tgaagacttt ctaaaaactc cacaagagat cacttcacat 8340
   ccttcacaca tgtcctcctt tgcacagttg gcaggtttca gacatataca tatatttcta 8400
   tgacaaggct tatcattctt taggactgca gtaatttaga taagaagctc tcaaaagaac 8460
   acttgcccag taacagcatc tccaccaatg aactgatgac agctctggct ctgagcctct 8520
   ggaaccaatg aactctgttc attgaacaga actcttgctt ctgtttccaa gcagctttat 8580
   ttgaacttgc cctttttgcc aataaaagct tccttttacc cttccctcac tggacgtact 8640
   tttggcttgc catttcgtgc atcctgaatt ctaatattct cttcccattt ctgaataaat 8700
   tcaacatatt tggagatatt tttctccagt cttttttttc ccttttttga gactgaaaga 8760
   ggtctgatgc ttaggtgcat attttttaac tatttagctg tattgagtct tagatgatgt 8820
   ttaccactaa aaaaaaaact aactccaaca tctggatcaa atgtgctagg gcaagcaagt 8880
   ctacactgtc catagattgt gacctcgttc tttaagaatc tgatgtgggt ggggcatggt 8940
   ggctcatacc tgtaatccga gcactttggg aggctgaggc aggaggacag cttgaggtca 9000
   ggagttcaag accagactag gcaacatagc aagaaccccc atctctagca aaaagtaaaa 9060
   aaacaaacaa aaaaaccaca cacatctggc catggtggca cgcacctgta gtctcagcta 9120
   ctcgggaggc tggggtagga ggatggcttg agcccaggag tttgaggttg cagtgagcta 9180
   tgaatgttgc cactgcactc cagcctgggt gaaaccctgt ctccaaaact aagctaaact 9240
   aaaaaaaaaa aaattctgat gtgactgtga ttttactagc tcaaacataa tgagctacaa 9300
   agtgagcaaa gttcactatg actacaaatg aaaagttgaa agaaatccat tgtcggcagg 9360
   gcgcggtggc tcacgcctgt aatcccagca ctttgggagg ctggggcggg tggatcacca 9420
   gaggtcagga gttcgagacc agcctgacca atatggtgaa accccgtctg tactaaaaat 9480
   acaaaaatta cctgggcatg gtgacaggca cctgtaatcc cagctactca ggaggctgag 9540
   gcaggagaat cgcttgagcc ggggaggctg aggttgcagt gaggcgagat cgtgccactg 9600
   cactccagcc tgggcaacag agcggaagga aggaagaaat gaaggaggga gggagggagg 9660
   gagggaggga accattatct gttgaatgcc tagtatgtgc cagaaagcca gggaaataaa 9720
   tgaaaagtgc aattggagct tgcaaaaagg aaggtcaaga tgctaaagaa ggtttagcgg 9780
   aaactgaact gggatttgat catattaatt aatatgaaca aaaagagaga aaagaatgta 9840
   gtttgtaggc caggtagaag aagaactaat tttatagaag aaaaacaaat ctaaatgtta 9900
   tatcttttat ttctcttgtc aaaaacttgt agtccaatta tttttaactc cttatgtatt 9960
   tccattttca gtctgtctac aaggaaaact gactcgaggt aattaaggat agagatcaag 10020
   gggcagattt tatttgtatg gtggtaaagt tcaaagggct ctttgctcca caatcgatcc 10080
   actaagacca aaaactttag gtgtactcta gagtgtcaca tccaagtatt taaaccatta 10140
   aatttgggtg gacaaagggg aggatgtgga aagggaaatg agataccaaa aactgataga 10200
   tgtttaaaaa agaggtagtt tggaaactac cttttttaag actgacaatt aggaagatac 10260
   atttttggca gtatatacca ctgtttcact ctaaaatcac cgttgaactg ttatgtaact 10320
   ttcatctatt tatttaatta ctgaatccat gcttcaacaa cccaagccta agtaacacca 10380
   aataaaggac tgagattgag gctttttttt tttttttttg agacaaggtc tcgctctgtc 10440
   gcccaggctg gagggcagtt cttcgctcac tacctctgcc tcctaggctc aagctgggac 10500
   tacagacgcg cgccgccccg cccaactaat tttttcttct tctttttttt tttttttttt 10560
   gagagtctcg ctctgtcgcc caggctggag tgcagtggca ctatctcggc tcactgcaac 10620
   ctccgtctcc caggttcaag cgattctcct gcctcagctt cccgggtagc tgggactaca 10680
   ggtgcgcgcc aacactctgt ctaatttttg tatttttagt agagacgggg tttcaccatg 10740
   ctggccaggc tggtctccaa ctcccgacct caggcgattc tcccgcctcg gcctcccaaa 10800
   gtgctgggat tacagtcgtg agccaccgcg cccggcctag ttaatttttt aatttaattt 10860
   ttttgtggag acgaggtctc actattgccc aggctaatct cgaacttctg ggctaagcga 10920
   tcctcctgcc tcgagcctcc caaattgttg ggcttacagg cgtgagccac cgcacctggc 10980
   ggaacatttc tactctaatg aaaattataa cagtttttac agttttccta gctcccaact 11040
   gtacctataa gttcagagtc catttaatgt acttgtaata attcaggctg gtgttttatt 11100
   atcttttcta cattattacc ctctcaaaga acaaaagcag gtaactccta taaagtctaa 11160
   tagtctgaaa attgtagtgt ccgttttctc tttagccata aagttttgct taaatgctga 11220
   gtcgcgtttt aatgctctac tagatgtaca tataagaata ctaaatggag ggaaagtaat 11280
   ttcttttttt tttttgagac ggggtcactc aggctggagt gccagtgcgc gattacagct 11340
   caccggagcc tctcgacctc cccaggctca agtgatcctg ccacctcagc ctccccaata 11400
   gctgggacta cagacacgcg ccaccacgcc cggctaattt ttttgtattt ttcttagaga 11460
   ctgggttttg ccatgttgcc caaactggtc ttgaactcct gggctcaagc gacctgcctg 11520
   cctcagcccc gcaaagtgct gggattacag gcgtgagcca ccgcgcccgg ccataatttt 11580
   atcttaatca ctttttccca ggaactatta cgtttcaaag aatgaggaat taactgacaa 11640
   cacttcaggt tcaacatgta aatagggacg ctcatttgag aataaatcac attttgtcac 11700
   agttgtattg aaaaatcggt atttctgaac gtcattttac cattacggaa gtataactca 11760
   gacacaataa aatgtttaat atacggctag gattaggtta aggtgagaga ggcgcctaga 11820
   gcacaaattt taaggaggca agaccacgac cccgaccgtg agcgcctgct taacatttgc 11880
   tccccaggcg cttcccactc cccagccggg ccccgtccta gttcaatcaa tcaagaaaat 11940
   caagagtacg aaggccttgt gcggtctgga cggtctttcc acgtctcagg tgcagttaag 12000
   ccagtcacac tccatccacc tacccgccat gaaaaattat ttgttcagca ggaattgcca 12060
   agtcaaatac agccatataa agcccccctt cgacgtgcca ggtgtgcctc cggagagcag 12120
   ccaccaaaac caccagcagc ccgtccgcgc cgggaaatgc gccctcgctg gcttacgggg 12180
   ccactcagac agttctaagt gcctttccct gtaacgcaga ggcacacctg ccttccctcg 12240
   ggaaacagcg gcccgacgtg cgcccgcaac gcgtaaagcg tcaacagctg aaaccctcgg 12300
   aatcctcccc gaacagaaac aaccaccgct gccagctgcg cgctcggggg aaaagacgtt 12360
   gcgcccccgc cgactgccgg tttcccgggc gcgagcccgg atccaggtgg tcagtcccgg 12420
   tacgcaacca cggcgagaac ccggccctgc taagggagaa gggaagccgt ttcccgcggg 12480
   cttcatacag acacggtgct aagacagccc gagtcgcgga ccagtccaac aggcaggcag    12540
   atcagtgggc agcccgcgtg c                                              12561


<210>  22
<211>  111
<212>  RNA
<213>  Artificial Sequence

<220>
<223>  guide RNA

<400>  22
gggcgugucu cggcaccccu guuucagagc uaugcuggaa acagcauagc aaguugaaau      60

aaggcuaguc cguuaucaac uugaaaaagu ggcaccgagu cggugcuuuu u              111


<210>  23
<211>  111
<212>  RNA
<213>  Artificial Sequence

<220>
<223>  guide RNA

<400>  23
gagcucccgg guuccggagc guuucagagc uaugcuggaa acagcauagc aaguugaaau      60

aaggcuaguc cguuaucaac uugaaaaagu ggcaccgagu cggugcuuuu u              111


<210>  24
<211>  111
<212>  RNA
<213>  Artificial Sequence

<220>
<223>  guide RNA

<400>  24
ggggcggcua cucgaguuca guuucagagc uaugcuggaa acagcauagc aaguugaaau      60

aaggcuaguc cguuaucaac uugaaaaagu ggcaccgagu cggugcuuuu u              111


<210>  25
<211>  111
<212>  RNA
<213>  Artificial Sequence

<220>
<223>  guide RNA

<400>  25
gaauuccggg ucaaugggcg guuucagagc uaugcuggaa acagcauagc aaguugaaau      60

aaggcuaguc cguuaucaac uugaaaaagu ggcaccgagu cggugcuuuu u              111


<210>  26
<211>  3411

<212>  DNA
<213>  Artificial Sequence

<220>
<223>  expression construct

<400>  26
gtggtggcgg gcgcctgtag tcccagctac tcgggactac aggagaatcg cttgaaccca      60

agaggcggag tgttgcagtg agccaagatc gctccactgc actccagtct ggcgacagag     120

cgagaccccg tcttaaaaaa aacaaaacaa aacaaaaaaa aaacctttgc tttcccgcct     180

tccaagagga aggggcgcat ctctgaggaa acaagcccgg ggaacccgca gcccggacgg     240

catcctctca tctacacaac ccgaggacat ctccctcagc tcccagagga cggcggacca     300

tggtcaaggt cctctttccg gcctttcaca actccaagcc ccgccccaca aaaccccctg     360

cagtcctacc aagtcactcc catggcgtcg ggccaggaag gtggcagaat cacacagtag     420

aaacgcaggg ttccctgtcc gcccacagaa actccttcag ttccccaaca caaccttctg     480

tctcagaagg gactccggaa atacgtaaaa acagagcgcc gggtccacgc cttccccaag     540

gaatggggcg cgtctcggca cctaggctag cgatcctcta gattcgagtt tactccctat     600

cagtgataga gaacgtatga agagtttact ccctatcagt gatagagaac gtatgcagac     660

tttactccct atcagtgata gagaacgtat aaggagttta ctccctatca gtgatagaga     720

acgtatgacc agtttactcc ctatcagtga tagagaacgt atctacagtt tactccctat     780

cagtgataga gaacgtatat ccagtttact ccctatcagt gatagagaac gtataagctt     840

taggcgtgta cggtgggcgc ctataaaagc agagctagct gcagtaacgc cattttgcaa     900

ggcatggaaa aataccaaac caagaataga gaagttcaga tcaagggcgg gtacatgaaa     960

atagctaacg ttgggccaaa caggatatct gcggtgagca gtttcggccc cggcccgggg    1020

ccaagaacag atggtcaccg cagtttcggc cccggcccga ggccaagaac agatggtccc    1080

cagatatggc ccaaccctca gcagtttctt aagacccatc agatgtttcc aggctccccc    1140

aaggacctga aatgaccctg cgccttattt gaattaacca atcagcctgc ttctcgcttc    1200

tgttcgcgcg cttctgcttc ccgagctcta taaaagagct cacaacccct cactcggcgc    1260

gccagtcctc cgactcgaga gcgctgccac catggtgagc aagggcgagg agctgttcac    1320

cggggtggtg cccatcctgg tcgagctgga cggcgacgta aacggccaca agttcagcgt    1380

gtccggcgag ggcgagggcg atgccaccta cggcaagctg accctgaagt tcatctgcac    1440

caccggcaag ctgcccgtgc cctggcccac cctcgtgacc accctgacct acggcgtgca    1500

gtgcttcagc cgctaccccg accacatgaa gcagcacgac ttcttcaagt ccgccatgcc    1560

cgaaggctac gtccaggagc gcaccatctt cttcaaggac gacggcaact acaagacccg    1620

cgccgaggtg aagttcgagg gcgacaccct ggtgaaccgc atcgagctga agggcatcga    1680

cttcaaggag gacggcaaca tcctggggca caagctggag tacaactaca acagccacaa 1740

cgtctatatc atggccgaca agcagaagaa cggcatcaag gtgaacttca agatccgcca 1800

caacatcgag gacggcagcg tgcagctcgc cgaccactac cagcagaaca cccccatcgg 1860

cgacggcccc gtgctgctgc ccgacaacca ctacctgagc acccagtccg ccctgagcaa 1920

agaccccaac gagaagcgcg atcacatggt cctgctggag ttcgtgaccg ccgccgggat 1980

cactctcggc atggacgagc tgtacaaggg atccggagcg actaacttct ccctgctgaa 2040

gcaggccggc gatgtagaag agaacccggg ccctaccgag tacaagccca cggtgcgcct 2100

cgccacccgc gacgacgtcc ccagggccgt acgcaccctc gccgccgcgt tcgccgacta 2160

ccccgccacg cgccacaccg tcgatccgga ccgccacatc gagcgggtca ccgagctgca 2220

agaactcttc ctcacgcgcg tcgggctcga catcggcaag gtgtgggtcg cggacgacgg 2280

cgccgccgtg gcggtctgga ccacgccgga gagcgtcgaa gcgggggcgg tgttcgccga 2340

gatcggcccg cgcatggccg agttgagcgg ttcccggctg gccgcgcagc aacagatgga 2400

aggcctcctg gcgccgcacc ggcccaagga gcccgcgtgg ttcctggcca ccgtcggagt 2460

ctcgcccgac caccagggca agggtctggg cagcgccgtc gtgctccccg gagtggaggc 2520

ggccgagcgc gccggggtgc ccgccttcct ggaaacctcc gcgccccgca acctcccctt 2580

ctacgagcgg ctcggcttca ccgtcaccgc cgacgtcgag gtgcccgaag gaccgcgcac 2640

ctggtgcatg acccgcaagc ccggtgcctg aggcacccct cgatgacgcc tccactgaca 2700

tcatctgcgg attcccgccc cgaggtttct aaatccagac attcccgttt ggctgagcac 2760

tctaggccta catccatgaa gctaggagag cgacactcaa agactgcact attgagagaa 2820

gctaacgtta aaggcagtga atatattcgg gagtacagct ccggaacgcg tgagctcttt 2880

tagtgggagg ggcggcgctg atggcgcttc tggcctccga atgctagggg gcgctgtgat 2940

cgccgggcgg cctcttgggc gctgggagtc caccgcgcaa gcgcatcctg gcctttcttc 3000

agtccccacg tgcgatcctt cccggcaact ttttcgagaa aaatgcccaa attcaaggcg 3060

gcccgtgggg tggggggtca ggaaaaacat gcgcccctgg ccgatcagat cctggctggg 3120

aatgcggtgc gggcgggggt ccgggagaag cggcggggtc gcgggacagg agaagcggag 3180

gaagagtatg tggggccccg gctgagccga cggattttgc agcaagcacg gcagcaacag 3240

gaggaactcg aggccgagca tgggactggg gacaagcccg cggcgccgcg ggaacgcacc 3300

acgcggctgg gtgagtgtct gggatgaggt ccgaggaaga cagtggccag tagtggggcg 3360

gggtgggcag ctaaaaagtg tctggcaggg gaattgcttc gagtcaacaa a 3411

<210> 27
<211> 3411
<212> DNA
<213> Artificial Sequence

<220>
<223>  expression construct

<400>  27
gtggtggcgg gcgcctgtag tcccagctac tcgggactac aggagaatcg cttgaaccca      60

agaggcggag tgttgcagtg agccaagatc gctccactgc actccagtct ggcgacagag     120

cgagaccccg tcttaaaaaa aacaaaacaa aacaaaaaaa aaacctttgc tttcccgcct     180

tccaagagga aggggcgcat ctctgaggaa acaagcccgg ggaacccgca gcccggacgg     240

catcctctca tctacacaac ccgaggacat ctccctcagc tcccagagga cggcggacca     300

tggtcaaggt cctctttccg gcctttcaca actccaagcc ccgccccaca aaaccccctg      360

cagtcctacc aagtcactcc catggcgtcg ggccaggaag gtggcagaat cacacagtag     420

aaacgcaggg ttccctgtcc gcccacagaa actccttcag ttccccaaca caaccttctg     480

tctcagaagg gactccggaa atacgtaaaa acagagcgcc gggtccacgc cttccccaag     540

gaatggggcg cgtctcggca cccctcgatg acgcctccac tgacatcatc tgcggattcc     600

cgccccgagg tttctaaatc cagacattcc cgtttggctg agcactctag gcctacatcc     660

atgaagctag gagagcgaca ctcaaagact gcactattga gagaagctaa cgttaaaggc     720

agtgaatata ttcgggagta cagctccgga acgcgctagg ctagcgatcc tctagattcg     780

agtttactcc ctatcagtga tagagaacgt atgaagagtt tactccctat cagtgataga     840

gaacgtatgc agactttact ccctatcagt gatagagaac gtataaggag tttactccct     900

atcagtgata gagaacgtat gaccagttta ctccctatca gtgatagaga acgtatctac     960

agtttactcc ctatcagtga tagagaacgt atatccagtt tactccctat cagtgataga    1020

gaacgtataa gctttaggcg tgtacggtgg gcgcctataa aagcagagct agctgcagta    1080

acgccatttt gcaaggcatg gaaaaatacc aaaccaagaa tagagaagtt cagatcaagg    1140

gcgggtacat gaaaatagct aacgttgggc caaacaggat atctgcggtg agcagtttcg    1200

gccccggccc ggggccaaga acagatggtc accgcagttt cggccccggc ccgaggccaa    1260

gaacagatgg tccccagata tggcccaacc ctcagcagtt tcttaagacc catcagatgt    1320

ttccaggctc ccccaaggac ctgaaatgac cctgcgcctt atttgaatta accaatcagc    1380

ctgcttctcg cttctgttcg cgcgcttctg cttcccgagc tctataaaag agctcacaac    1440

ccctcactcg gcgcgccagt cctccgactc gagagcgctg ccaccatggt gagcaagggc    1500

gaggagctgt tcaccggggt ggtgcccatc ctggtcgagc tggacggcga cgtaaacggc    1560

cacaagttca gcgtgtccgg cgagggcgag ggcgatgcca cctacggcaa gctgaccctg    1620

aagttcatct gcaccaccgg caagctgccc gtgccctggc ccaccctcgt gaccaccctg    1680

acctacggcg tgcagtgctt cagccgctac cccgaccaca tgaagcagca cgacttcttc    1740

aagtccgcca tgcccgaagg ctacgtccag gagcgcacca tcttcttcaa ggacgacggc 1800

aactacaaga cccgcgccga ggtgaagttc gagggcgaca ccctggtgaa ccgcatcgag 1860

ctgaagggca tcgacttcaa ggaggacggc aacatcctgg ggcacaagct ggagtacaac 1920

tacaacagcc acaacgtcta tatcatggcc gacaagcaga agaacggcat caaggtgaac 1980

ttcaagatcc gccacaacat cgaggacggc agcgtgcagc tcgccgacca ctaccagcag 2040

aacaccccca tcggcgacgg ccccgtgctg ctgcccgaca accactacct gagcacccag 2100

tccgccctga gcaaagaccc caacgagaag cgcgatcaca tggtcctgct ggagttcgtg 2160

accgccgccg ggatcactct cggcatggac gagctgtaca agggatccgg agcgactaac 2220

ttctccctgc tgaagcaggc cggcgatgta gaagagaacc cgggccctac cgagtacaag 2280

cccacggtgc gcctcgccac ccgcgacgac gtccccaggg ccgtacgcac cctcgccgcc 2340

gcgttcgccg actaccccgc cacgcgccac accgtcgatc cggaccgcca catcgagcgg 2400

gtcaccgagc tgcaagaact cttcctcacg cgcgtcgggc tcgacatcgg caaggtgtgg 2460

gtcgcggacg acggcgccgc cgtggcggtc tggaccacgc cggagagcgt cgaagcgggg 2520

gcggtgttcg ccgagatcgg cccgcgcatg gccgagttga gcggttcccg gctggccgcg 2580

cagcaacaga tggaaggcct cctggcgccg caccggccca aggagcccgc gtggttcctg 2640

gccaccgtcg gagtctcgcc cgaccaccag ggcaagggtc tgggcagcgc cgtcgtgctc 2700

cccggagtgg aggcggccga gcgcgccggg gtgcccgcct tcctggaaac ctccgcgccc 2760

cgcaacctcc ccttctacga gcggctcggc ttcaccgtca ccgccgacgt cgaggtgccc 2820

gaaggaccgc gcacctggtg catgacccgc aagcccggtg cctgagcgcg tgagctcttt 2880

tagtgggagg ggcggcgctg atggcgcttc tggcctccga atgctagggg gcgctgtgat 2940

cgccgggcgg cctcttgggc gctgggagtc caccgcgcaa gcgcatcctg gcctttcttc 3000

agtccccacg tgcgatcctt cccggcaact ttttcgagaa aaatgcccaa attcaaggcg 3060

gcccgtgggg tggggggtca ggaaaaacat gcgcccctgg ccgatcagat cctggctggg 3120

aatgcggtgc gggcgggggt ccgggagaag cggcggggtc gcgggacagg agaagcggag 3180

gaagagtatg tggggccccg gctgagccga cggattttgc agcaagcacg gcagcaacag 3240

gaggaactcg aggccgagca tgggactggg gacaagcccg cggcgccgcg ggaacgcacc 3300

acgcggctgg gtgagtgtct gggatgaggt ccgaggaaga cagtggccag tagtggggcg 3360

gggtgggcag ctaaaaagtg tctggcaggg gaattgcttc gagtcaacaa a 3411

<210> 28
<211> 3393
<212> DNA
<213> Artificial Sequence

<220>

<223>  expression construct

<400>  28
tccggcctcg caaccgacca ccccttctgc aggagaggta cataatggtc gcaaagagca      60

ggcgaaccgg gcagagatcg ctcgctcgct cgcagactga gcgaatccgg actcacccgt     120

ctccttcgcc aagtgataaa acttgtctcg tcggctcttg ccaactttgc ccttcttgcc     180

catggtggaa agggggagta tccctcaatc tggggagaaa gtagaagttg ggaacgtctc     240

tttccggagc accagattcc acttccagtt tcccacttcc agtttcccac ttcccgcagc     300

aattccaccc cgcgcccctc tccgcacact acctagaccc agagccgctt tctccacact     360

tggaaccgca caagtatgca gctaactact tccgcttccg cttgcgtccc ctgcgtccct     420

ggctcgaggt tttccgccat tttgagtgtg gccctagatt gttctcaata ctctgtcctt     480

gggttttgta agttgaaagt tgagactagg aaggcatatc acaagaacta taaacagagt     540

ttcaatgagg caacactgag gaggagttct actccttcct catccccttc caaagcacct     600

gaactctagc taggctagcg atcctctaga ttcgagttta ctccctatca gtgatagaga     660

acgtatgaag agtttactcc ctatcagtga tagagaacgt atgcagactt tactccctat     720

cagtgataga gaacgtataa ggagtttact ccctatcagt gatagagaac gtatgaccag     780

tttactccct atcagtgata gagaacgtat ctacagttta ctccctatca gtgatagaga     840

acgtatatcc agtttactcc ctatcagtga tagagaacgt ataagcttta ggcgtgtacg     900

gtgggcgcct ataaaagcag agctagctgc agtaacgcca ttttgcaagg catggaaaaa     960

taccaaacca agaatagaga agttcagatc aagggcgggt acatgaaaat agctaacgtt    1020

gggccaaaca ggatatctgc ggtgagcagt ttcggccccg gcccggggcc aagaacagat    1080

ggtcaccgca gtttcggccc cggcccgagg ccaagaacag atggtcccca gatatggccc    1140

aaccctcagc agtttcttaa gacccatcag atgtttccag gctcccccaa ggacctgaaa    1200

tgaccctgcg ccttatttga attaaccaat cagcctgctt ctcgcttctg ttcgcgcgct    1260

tctgcttccc gagctctata aaagagctca caacccctca ctcggcgcgc cagtcctccg    1320

actcgagagc gctgccacca tggtgagcaa gggcgaggag ctgttcaccg gggtggtgcc    1380

catcctggtc gagctggacg gcgacgtaaa cggccacaag ttcagcgtgt ccggcgaggg    1440

cgagggcgat gccacctacg gcaagctgac cctgaagttc atctgcacca ccggcaagct    1500

gcccgtgccc tggcccaccc tcgtgaccac cctgacctac ggcgtgcagt gcttcagccg    1560

ctaccccgac cacatgaagc agcacgactt cttcaagtcc gccatgcccg aaggctacgt    1620

ccaggagcgc accatcttct tcaaggacga cggcaactac aagacccgcg ccgaggtgaa    1680

gttcgagggc gacaccctgg tgaaccgcat cgagctgaag ggcatcgact tcaaggagga    1740

cggcaacatc ctggggcaca agctggagta caactacaac agccacaacg tctatatcat    1800

ggccgacaag cagaagaacg gcatcaaggt gaacttcaag atccgccaca acatcgagga 1860

cggcagcgtg cagctcgccg accactacca gcagaacacc cccatcggcg acggccccgt 1920

gctgctgccc gacaaccact acctgagcac ccagtccgcc ctgagcaaag accccaacga 1980

gaagcgcgat cacatggtcc tgctggagtt cgtgaccgcc gccgggatca ctctcggcat 2040

ggacgagctg tacaagggat ccggagcgac taacttctcc ctgctgaagc aggccggcga 2100

tgtagaagag aacccgggcc ctaccgagta caagcccacg gtgcgcctcg ccacccgcga 2160

cgacgtcccc agggccgtac gcaccctcgc cgccgcgttc gccgactacc ccgccacgcg 2220

ccacaccgtc gatccggacc gccacatcga gcgggtcacc gagctgcaag aactcttcct 2280

cacgcgcgtc gggctcgaca tcggcaaggt gtgggtcgcg gacgacggcg ccgccgtggc 2340

ggtctggacc acgccggaga gcgtcgaagc gggggcggtg ttcgccgaga tcggcccgcg 2400

catggccgag ttgagcggtt cccggctggc cgcgcagcaa cagatggaag gcctcctggc 2460

gccgcaccgg cccaaggagc ccgcgtggtt cctggccacc gtcggagtct cgcccgacca 2520

ccagggcaag ggtctgggca gcgccgtcgt gctccccgga gtggaggcgg ccgagcgcgc 2580

cggggtgccc gccttcctgg aaacctccgc gccccgcaac ctccccttct acgagcggct 2640

cggcttcacc gtcaccgccg acgtcgaggt gcccgaagga ccgcgcacct ggtgcatgac 2700

ccgcaagccc ggtgcctgag ctagaagccg cccctcccat catggttccc ttagtgtggt 2760

ctcgccgcac aacccgccca tgtacacgga attcttctct gcctggtctt caggtcccaa 2820

tcctccgctt ccgcgcttgc gcgccaagac ggctcggatg ccggcggtct ctgctgaaga 2880

gagaagatgg cgcttgacgg accagagcag gtatggcggg tgcagtggcg gcccggcagg 2940

ttacggggct gggtgcggag cgagcgtgat ctgagtggag agcgggccgg ggcaggagcg 3000

tcgggtgggt cggaggtgcc tggaccagtc catgctggac gctgcctgcg accggatctg 3060

ctgtgtcagc gccgccctcg gtgagtcagg gcaaggctgg gagaggaaga cgcggtagaa 3120

gcggagtgag tgagggaagc gatgggcgcg ggaatggccg gcccacgggt cgcaggagac 3180

gggacgccag tcctttggct ccgttccgct ggctccttcg tcagtactga cacctcgggc 3240

ttgtagagca cttcacgcag tgcaaagcgc cccccgtcta tatcatatcg cctctcggtc 3300

ctcctaaaag tcgtatgagg taggcggcgt tcccattctt ttattttagt cctgggaaaa 3360

tggagggttc caagggaggg agggattaga acc 3393

<210> 29
<211> 3393
<212> DNA
<213> Artificial Sequence

<220>
<223> expression construct

<400> 29
tccggcctcg caaccgacca ccccttctgc aggagaggta cataatggtc gcaaagagca 60

ggcgaaccgg gcagagatcg ctcgctcgct cgcagactga gcgaatccgg actcacccgt 120

ctccttcgcc aagtgataaa acttgtctcg tcggctcttg ccaactttgc ccttcttgcc 180

catggtggaa agggggagta tccctcaatc tggggagaaa gtagaagttg ggaacgtctc 240

tttccggagc accagattcc acttccagtt tcccacttcc agtttcccac ttcccgcagc 300

aattccaccc cgcgcccctc tccgcacact acctagaccc agagccgctt tctccacact 360

tggaaccgca caagtatgca gctaactact tccgcttccg cttgcgtccc ctgcgtccct 420

ggctcgaggt tttccgccat tttgagtgtg gccctagatt gttctcaata ctctgtcctt 480

gggttttgta agttgaaagt tgagactagg aaggcatatc acaagaacta taaacagagt 540

ttcaatgagg caacactgag gaggagttct actccttcct catccccttc caaagcacct 600

gaactctaga agccgcccct cccatcatgg ttcccttagt gtggtctcgc cgcacaaccc 660

gcccatgtac ctaggctagc gatcctctag attcgagttt actccctatc agtgatagag 720

aacgtatgaa gagtttactc cctatcagtg atagagaacg tatgcagact ttactcccta 780

tcagtgatag agaacgtata aggagtttac tccctatcag tgatagagaa cgtatgacca 840

gtttactccc tatcagtgat agagaacgta tctacagttt actccctatc agtgatagag 900

aacgtatatc cagtttactc cctatcagtg atagagaacg tataagcttt aggcgtgtac 960

ggtgggcgcc tataaaagca gagctagctg cagtaacgcc attttgcaag gcatggaaaa 1020

ataccaaacc aagaatagag aagttcagat caaggcgggt acatgaaaa tagctaacgt 1080

tgggccaaac aggatatctg cggtgagcag tttcggcccc ggcccggggc caagaacaga 1140

tggtcaccgc agtttcggcc ccggcccgag gccaagaaca gatggtcccc agatatggcc 1200

caaccctcag cagtttctta agacccatca gatgtttcca ggctccccca aggacctgaa 1260

atgaccctgc gccttatttg aattaaccaa tcagcctgct tctcgcttct gttcgcgcgc 1320

ttctgcttcc cgagctctat aaaagagctc acaacccctc actcggcgcg ccagtcctcc 1380

gactcgagag cgctgccacc atggtgagca agggcgagga gctgttcacc ggggtggtgc 1440

ccatcctggt cgagctggac ggcgacgtaa acggccacaa gttcagcgtg tccggcgagg 1500

gcgagggcga tgccacctac ggcaagctga ccctgaagtt catctgcacc accggcaagc 1560

tgcccgtgcc ctggcccacc ctcgtgacca ccctgaccta cggcgtgcag tgcttcagcc 1620

gctaccccga ccacatgaag cagcacgact tcttcaagtc cgccatgccc gaaggctacg 1680

tccaggagcg caccatcttc ttcaaggacg acggcaacta caagacccgc gccgaggtga 1740

agttcgaggg cgacaccctg gtgaaccgca tcgagctgaa gggcatcgac ttcaaggagg 1800

acggcaacat cctggggcac aagctggagt acaactacaa cagccacaac gtctatatca 1860

tggccgacaa gcagaagaac ggcatcaagg tgaacttcaa gatccgccac aacatcgagg 1920

acggcagcgt gcagctcgcc gaccactacc agcagaacac ccccatcggc gacggccccg 1980

tgctgctgcc cgacaaccac tacctgagca cccagtccgc cctgagcaaa gaccccaacg 2040

agaagcgcga tcacatggtc ctgctggagt tcgtgaccgc cgccgggatc actctcggca 2100

tggacgagct gtacaaggga tccggagcga ctaacttctc cctgctgaag caggccggcg 2160

atgtagaaga gaacccgggc cctaccgagt acaagcccac ggtgcgcctc gccacccgcg 2220

acgacgtccc cagggccgta cgcaccctcg ccgccgcgtt cgccgactac cccgccacgc 2280

gccacaccgt cgatccggac cgccacatcg agcgggtcac cgagctgcaa gaactcttcc 2340

tcacgcgcgt cgggctcgac atcggcaagg tgtgggtcgc ggacgacggc gccgccgtgg 2400

cggtctggac cacgccggag agcgtcgaag cgggggcggt gttcgccgag atcggcccgc 2460

gcatggccga gttgagcggt tcccggctgg ccgcgcagca acagatggaa ggcctcctgg 2520

cgccgcaccg gcccaaggag cccgcgtggt tcctggccac cgtcggagtc tcgcccgacc 2580

accagggcaa gggtctgggc agcgccgtcg tgctccccgg agtggaggcg gccgagcgcg 2640

ccggggtgcc cgccttcctg gaaacctccg cgccccgcaa cctccccttc tacgagcggc 2700

tcggcttcac cgtcaccgcc gacgtcgagg tgcccgaagg accgcgcacc tggtgcatga 2760

cccgcaagcc cggtgcctga ggtacacgga attcttctct gcctggtctt caggtcccaa 2820

tcctccgctt ccgcgcttgc gcgccaagac ggctcggatg ccggcggtct ctgctgaaga 2880

gagaagatgg cgcttgacgg accagagcag gtatggcggg tgcagtggcg gcccggcagg 2940

ttacggggct gggtgcggag cgagcgtgat ctgagtggag agcgggccgg ggcaggagcg 3000

tcgggtgggt cggaggtgcc tggaccagtc catgctggac gctgcctgcg accggatctg 3060

ctgtgtcagc gccgccctcg gtgagtcagg gcaaggctgg gagaggaaga cgcggtagaa 3120

gcggagtgag tgagggaagc gatgggcgcg ggaatggccg gcccacgggt cgcaggagac 3180

gggacgccag tcctttggct ccgttccgct ggctccttcg tcagtactga cacctcgggc 3240

ttgtagagca cttcacgcag tgcaaagcgc cccccgtcta tatcatatcg cctctcggtc 3300

ctcctaaaag tcgtatgagg taggcggcgt tcccattctt ttattttagt cctgggaaaa 3360

tggagggttc caaggagggg agggattaga acc 3393

<210> 30
<211> 335
<212> PRT
<213> Artificial Sequence

<220>
<223> TetR-KRAB

<400> 30

Met Ala Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Ser Pro
        195                 200                 205

Lys Lys Lys Arg Lys Val Asp Gly Gly Gly Ala Leu Ser Pro Gln His
    210                 215                 220

Ser Ala Val Thr Gln Gly Ser Ile Ile Lys Asn Lys Glu Gly Met Asp
225                 230                 235                 240

Ala Lys Ser Leu Thr Ala Trp Ser Arg Thr Leu Val Thr Phe Lys Asp
                245                 250                 255

Val Phe Val Asp Phe Thr Arg Glu Glu Trp Lys Leu Leu Asp Thr Ala
            260                 265                 270

Gln Gln Ile Val Tyr Arg Asn Val Met Leu Glu Asn Tyr Lys Asn Leu
        275                 280                 285

Val Ser Leu Gly Tyr Gln Leu Thr Lys Pro Asp Val Ile Leu Arg Leu
    290                 295                 300

Glu Lys Gly Glu Glu Pro Trp Leu Val Glu Arg Glu Ile His Gln Glu
305                 310                 315                 320

Thr His Pro Asp Ser Glu Thr Ala Phe Glu Ile Lys Ser Ser Val
                325                 330                 335

## Description

### FIELD OF THE INVENTION

The invention refers to relates to a recombinant host cell and methods of expressing a gene of interest (GOI) from a host cell. The invention relates particularly to methods of improving a host cell's capacity to stably express a GOI over a long period of time, and gene therapy employing the recombinant host cell.

### BACKGROUND OF THE INVENTION

Cell and gene therapy relies on the insertion of transgenes in target cells. Insertion can happen in many different ways, using for instance transposons or lentiviral vectors. Transgene insertion may also happen via homology-directed repair, facilitated by programmable nucleases such as Zinc finger nucleases (ZFNs), transcriptional activator-like effector nucleases (TALENs), Clustered Regularly Interspaced Short Palindromic Repeats (CRIPSRs), homing endonucleases and meganucleases. Popular transgenes include genes whose function is defective, but also chimeric antigen receptors (for CAR-T therapy), T cell receptors (for TCR therapy) or cDNAs whose function is defective in patients.

Stable expression in eukaryotic cell lines is useful for numerous projects. For example, it supports recombinant protein production over a prolonged period in a cell culture, or it helps in transgenic eukaryotes for optimization of protein productions at high yields. Maintaining stable expression of transgenes is important to ensure functionality. Stable expression implies insertion into the genome.

Randomly inserted genes are subject to position effects and silencing, making their expression unreliable and unpredictable. Targeted insertion in safe-harbor sites has made some progress. To this end, so called safe harbour loci can be targeted e.g., *AAVS1* in human (also known as *PPP1R12C*) locus, a well-validated "safe harbor" in the human genome, or *ROSA26* in mouse, that are actively transcribed.

Safe harbor sites are defined based on their position relative to contiguous coding genes, microRNAs and ultra-conserved regions and, for example understood to meet the following criteria: (i) distance of at least 50 kb from the 5' end of any gene, (ii) distance of at least 300 kb from any cancer-related gene, (iii) distance of at least 300 kb from any microRNA (miRNA), (iv) location outside a transcription unit and (v) location outside ultra-conserved regions (UCRs) of the human genome (Bejerano G, et al.. Science. 2004;304:1321-1325; Pellenz S, et al., Hum Gene Ther. 2019;30(7):814-828).

Nevertheless, transgene silencing remains an important problem that is not easily addressed and that hampers the feasibility of the above-mentioned approaches. Silencing is a phenomenon that is implemented by establishing repressive marks on DNA or histones e.g. trimethylation of histone H3 on lysine 9 or 27. When silencing occurs, these repressive chromatin marks are spread over a certain region, typically at least several kilobases of DNA sequence (Mlambo et al. Nucleic Acids Res. 2018; 46(9):4456-4468) and may not only affect the transgene itself, but also adjacent regions.

### SUMMARY OF THE INVENTION

It is the objective to overcome transgene silencing by selecting safe harbour loci in the genome that cannot be silenced. Another objective of the invention is to provide transgenic host cells with improved gene expression capabilities over a prolonged period of time.

The object is solved by the subject matter as claimed.

The invention provides for an isolated mammalian host cell comprising a heterologous gene of interest (GOI) chromosomally integrated at a target site within an intergenic region between a pair of adjacent essential genes.

Chromosomal integration is specifically obtained by one or more genetic modifications of the host cell chromosome within the intergenic region and/or at the target site.

Specifically, said one or more genetic modifications comprise an insertion or knock-in of the GOI or an expression construct comprising the GOI.

Specifically, the host cell is a diploid cell (which term is herein understood to encompass a near-diploid cell), such as containing two copies of each chromosome.

Specifically, the isolated host cell is provided in a host cell culture or provided as a pharmaceutical preparation or a donor cell for cell therapy.

Specifically, the adjacent essential genes are contiguous coding genes only separated by an intergenic region, and/or positioned such that the intergenic region between the two adjacent essential genes consists of a contiguous non-coding nucleotide sequence.

Specifically, the intergenic region described herein is understood to comprise or consist of a genomic safe harbor. Specifically, the safe harbor comprises a target site or locus for GOI insertion. The target site can be a randomly chosen site within the intergenic region, or a predetermined site.

Therefore, the intergenic region as described herein is understood to be the polynucleotide sequence between the adjacent essential genes, before a transgene (e.g. the GOI or an expression construct comprising the GOI) is inserted at the target site. In other words, the intergenic region is understood to comprise a certain contiguous sequence which is positioned between the adjacent essential genes that does not comprise a transgene. Where the cell is engineered to comprise a transgene within the intergenic region, the intergenic region is understood to comprise or consist of the polynucleotide sequence which identifies the region between the adjacent essential genes without the transgene, i.e. comprising or composed of two parts of the intergenic region flanking the 5'-end and 3'-end of the transgene, which parts are fused together, thereby obtaining a contiguous sequence comprising or composed of the nucleotide sequence of the flanking parts.

Specifically, the length of the intergenic region is less than any one of 20.000, 15.000, 10.000, 5.000, 2.500, or 1.000 nt.

Specifically, the intergenic region does not comprise a gene or part of a gene.

Specifically, the intergenic region is non-coding.

Specifically, at least any one of 60%, 70%, 80%, 85%, 90%, or 95% of the intergenic region consists of a polynucleotide of human origin, such as a human polynucleotide, Specifically, the intergenic region comprises a contiguous part of any one of SEQ ID NO:1-21, which is at least any one of 60%, 70%, 80%, 85%, 90%, or 95% of the full-length of the respective sequence of SEQ ID NO:1-21.

Specifically, the intergenic region comprises or consists of the nucleic acid sequence which is at least 90% identical to any one of SEQ ID NO:1-21.

According to a specific embodiment, the intergenic region comprises or consists of a polynucleotide which is of human origin, such as a human polynucleotide which is 100% identical to any one of SEQ ID NO:1-21.

Specifically, the intergenic region comprises or consists of a polynucleotide which is a naturally-occurring mammalian or human variant of any one of SEQ ID NO:1-21, such as e.g. a polynucleotide identified by any one of SEQ ID NO:1-21, wherein the polynucleotide sequence is modified by one or more single-nucleotide polymorphisms that naturally-occur within the polynucleotide sequence in the human genome.

Specifically, each of the essential genes is of human origin and/or has at least any one of 85%, 90%, 95%, 96%, 97%, 98%, 99% sequence identity to the respective human gene sequence, or is 100% to the respective human gene sequence.

Specifically, the essential genes are human genes or their natural variants originating from different human beings, or their analogs originating from different mammalian species, such as mouse, hamster, rat, dog or pig. The sequence of an essential gene is largely conserved, with a sequence identity of at least 80% or 85% or 90% of corresponding genes in non-human animals as compared to human beings. For example, the nucleotide sequence of MED20 from human is 93% identical to the corresponding gene in hamster (CHO), 90% identical to the corresponding gene in mouse, 99% identical to the corresponding gene in dog, and 98% identical to the corresponding gene in pig.

Specifically, each of the essential genes of said pair of essential genes is known to express proteins at a high expression level. Essential genes have core functions in the cell and are often highly conserved between species. They are defined as genes whose inactivation or deletion results in the death of the cell.

Specifically, the pair of essential genes is a pair consisting of a first and a second gene, wherein
i) the first gene is NFS1 and the second gene is ROMO1; or
ii) the first gene is MED22 and the second gene is RPLA7; or
iii) the first gene is DDX51 and the second gene is NOC4L; or
iv) the first gene is CENPK and the second gene is PPWD1; or
v) the first gene is ORC1 and the second gene is PRPF38A; or
vi) the first gene is POLR3K and the second gene is SNRNP25; or
vii) the first gene is COPE and the second gene is DDX49; or
viii) the first gene is FTSJ3 and the second gene is PSMC5; or
ix) the first gene is MED20 and the second gene is BYSL; or
x) the first gene is AURKA and the second gene is CSTF1; or
xi) the first gene is NUP88 and the second gene is RPAIN; or
xii) the first gene is ATP6V1D and the second gene is EIF2S1; or
xiii) the first gene is POLR2I and the second gene is TBCB; or
xiv) the first gene is UFD1 and the second gene is CDC45; or
xv) the first gene is CCDC115 and the second gene is IMP4; or
xvi) the first gene is NAA50 and the second gene is ATP6V1A; or
xvii) the first gene is SART3 and the second gene is ISCU; or
xviii) the first gene is C1orf109 and the second gene is CDCA8; or
xix) the first gene is POLR3A and the second gene is RPS24; or
xx) the first gene is RPS16 and the second gene is SUPT5H; or
xi) the first gene is RPS29 and the second gene is LRR1.

Each of the essential genes referred to herein is characterized by an Entrez Gene ID number. Entrez Gene is National Center for Biotechnology Information (NCBI)'s database for gene-specific information generating unique integers (GeneID) as stable identifiers for genes (Maglott et al. Nucleic Acids Res. 2011; 39 (Database issue):D52-D57). Each of the genes has a transcription start site (TSS) which denotes the start of transcription initiated by the promoter of the respective gene. The TSS in a nucleotide sequence can be identified using public databases such as RefSeq or FANTOM.

Specifically, the pair of essential genes and the respective intergenic region is characterized as shown in Table 1.

**Table 1: Exemplary pairs of essential genes and intergenic regions comprising a target site.**

| **Chr** | **gene1** | **GeneID gene1** | **TSS gene1** | **gene2** | **GeneID gene2** | **TSS gene2** | **Intergenic Region, nt*** | **Intergenic region start** | **Intergenic region end** | **SEQ ID NO** |
|---|---|---|---|---|---|---|---|---|---|---|
| chr20 | NFS1 | 9054 | 35699348 | ROMO1 | 140823 | 35699401 | 52 | 35699349 | 35699400 | 1 |
| chr9 | MED22 | 6837 | 133348090 | RPL7A | 6130 | 133348210 | 119 | 133348091 | 133348209 | 2 |
| chr12 | DDX51 | 317781 | 132144324 | NOC4L | 79050 | 132144450 | 125 | 132144325 | 132144449 | 3 |
| chr5 | CENPK | 64105 | 65563146 | PPWD1 | 23398 | 65563288 | 141 | 65563147 | 65563287 | 4 |
| chr1 | ORC1 | 4998 | 52404410 | PRPF38A | 84950 | 52404606 | 195 | 52404411 | 52404605 | 5 |
| chr16 | POLR3K | 51728 | 53618 | SNRNP25 | 79622 | 53858 | 239 | 53619 | 53857 | 6 |
| chr19 | COPE | 11316 | 18919374 | DDX49 | 54555 | 18919716 | 341 | 18919375 | 18919715 | 7 |
| chr17 | FTSJ3 | 117246 | 63827092 | PSMC5 | 5705 | 63827436 | 343 | 63827093 | 63827435 | 8 |
| chr6 | MED20 | 9477 | 41921128 | BYSL | 705 | 41921500 | 371 | 41921129 | 41921499 | 9 |
| chr20 | AURKA | 6790 | 56392204 | CSTF1 | 1477 | 56392646 | 441 | 56392205 | 56392645 | 10 |
| chr17 | NUP88 | 4927 | 5419662 | RPAIN | 84268 | 5420190 | 527 | 5419663 | 5420189 | 11 |
| chr14 | ATP6V1D | 51382 | 67359791 | EIF2S1 | 1965 | 67360338 | 546 | 67359792 | 67360337 | 12 |
| chr19 | POLR21 | 5438 | 36114890 | TBCB | 1155 | 36115502 | 611 | 36114891 | 36115501 | 13 |
| chr22 | UFD1 | 7353 | 19479184 | CDC45 | 8318 | 19479921 | 736 | 19479185 | 19479920 | 14 |
| chr2 | CCDC115 | 84317 | 130342152 | IMP4 | 92856 | 130342890 | 737 | 130342153 | 130342889 | 15 |
| chr3 | NAA50 | 80218 | 113746236 | ATP6V1A | 523 | 113747050 | 813 | 113746237 | 113747049 | 16 |
| chr12 | SART3 | 9733 | 108561169 | ISCU | 23479 | 108562603 | 1433 | 108561170 | 108562602 | 17 |
| chr1 | C1orf109 | 54955 | 37690515 | CDCA8 | 55143 | 37692536 | 2020 | 37690516 | 37692535 | 18 |
| chr10 | POLR3A | 11128 | 78029506 | RPS24 | 6229 | 78033862 | 4357 | 78029507 | 78033863 | 19 |
| chr19 | RPS16 | 6217 | 39435944 | SUPT5H | 6829 | 39445593 | 9648 | 39435945 | 39445592 | 20 |
| chr14 | RPS29 | 6235 | 49586376 | LRR1 | 122769 | 49598938 | 12561 | 49586377 | 49598937 | 21 |

| | | | | | | | | | | |
|---|---|---|---|---|---|---|---|---|---|---|
| * Between the TSS of gene 1 and the TSS of gene 2 | | | | | | | | | | |

According to a specific aspect, regulatory sequences selected from expression control sequences are employed, such as a promoter, an operator sequence, a transcriptional enhancer sequence or a transcriptional silencer sequence; or nucleotide sequences encoding expression factors, such as enhancers or inhibitors. Specifically, one or more regulatory sequences e.g., a promoter, are located between 2.000 or 1.000 bp bp upstream and 100 bp downstream of TSS of said GOI, in some cases of a small intergenic region, e.g. between any one of 1.000, 5.000, 1.000, 900, 800, 700, 600, 500, 400, 300, 200, 150, 100, or 50 bp upstream, and any one of 50 or 100 bp downstream a TSS.

Specifically, the transcription of the pair of the essential genes is in opposite orientation.

Specifically, each of the genes of a pair of essential genes is under transcriptional control of a respective essential gene promoter (EGP), preferably the EGP naturally-occurring with the respective gene, being either constitutive or inducible.

Specifically, the genes of said pair of essential genes are oriented such that their EGPs are adjacent and face in opposite directions.

The respective promoter regions operably linked to the first and the second genes of a pair of essential genes are preferably adjacent, or in close proximity, such that the distance between the respective TSSs is less than any one of 20.000, 15.000, 10.000, 5.000, 2.500, or 1.000 nt.

Specifically, the intergenic region comprising the target site comprises or consists of the region between the respective TSSs. Preferably, the target site is comprised in such region between the TSSs where the two essential genes are encoded on opposite strands and the TSSs are in close proximity..

According to a specific embodiment, the target site is at least partly or fully comprised within a promoter region. For example, a transgene is inserted within a region that is considered a promoter of one of the two essential genes, in particular where it is proven that the essential gene function is unaffected by the insertion of the transgene.

Specifically, the intergenic region is the region between and spanning the transcriptional start sites of the EGPs.

Specifically, the essential genes are so called "core essential genes", i.e. they are essential in many or all cell types or cell states studied to date. Consequently, their function needs to be maintained in all cell types and cell states of a certain species. For example, human core essential genes are considered essential in all human cell lines. Core essential genes may be determined by a genome-wide CRISPR/SpCas9 knockout screens, such as described by Hart el al. (G3: Genes, Genomes, Genetics August 1, 2017 vol. 7 no. 8 2719-2727) or Wang et al. (Science. 2014; 343(6166):80-84). Exemplary human core essential genes are listed in Table 1 below (as gene 1 and/or gene 2).

According to a specific aspect, the GOI is under transcriptional control of a heterologous promoter. The GOI can be integrated at the target site in a forward or reverse orientation.

Specifically, the GOI is comprised in a heterologous expression cassette integrated at the target site. The expression cassette may comprise at least one of a control element, promoter or regulatory element operatively linked to the GOI.

Specifically, the GOI is a heterologous gene or a transgene.

Specifically, the GOI is selected from any one of: a nucleic acid, an inhibitor, a cDNA or gene encoding a peptide or polypeptide, and a cDNA or gene encoding an antibody or antibody fragment, fusion protein, antigen, antagonist, agonist, RNAi molecule, or miRNA.

The target site is specifically understood as an insertion site, to insert the GOI or the expression cassette comprising the GOI.

The target site can comprise at least one or two sites (i.e. at least one or two locations) for the insertion of one or more GOIs (or expression constructs comprising a GOI) within the intergenic region at the target site. The intergenic region may be naturally-occurring or originating from a naturally occurring sequence and further be modified e.g. to incorporate a recognition target site or any other heterologous means to prepare for the knock-in of a transgene.

Besides insertion of the GOI (or the expression cassette comprising the GOI) at the target site, the intergenic region may be further modified by one or more mutations, such as deletion, insertion, or substitution of one or more nucleotides, which may be only a few e.g. up to 20nt, or up to 15nt, or up to 10nt, but may also change the nucleotide sequence such that the modified intergenic region (excluding the GOI sequence or a heterologous expression cassette) has at least any one of 50%, 60%, 70%, 80%, 85%, 90%, 95%, 98%, or 99% sequence identity, or is 100% identical to the naturally occurring intergenic region.

The exemplary intergenic regions characterized by any one of SEQ ID NO:1-21 may or may not harbor one or more single-nucleotide polymorphisms, in particular those which are naturally-occurring. Specifically, the intergenic region is a naturally-occurring intergenic region which may differ from any one of SEQ ID NO:1-21 by one or more single-nucleotide polymorphisms, or which are identical to any one of SEQ ID NO:1-21 and devoid of any single-nucleotide polymorphisms.

Exemplary host cells are stem cells including e.g. a hematopoietic stem cell, an embryonic stem cell, a pluripotent stem cell, or an endothelium cell.

According to a specific aspect, the host cell is a primary cell or an immune cell, such as of a cell type selected from the group consisting of a a Natural Killer (NK) cell, or a T cell, such as a cytotoxic T lymphocyte (CTL), a regulatory T cell or a T helper cell. Such host cell is specifically used in a pharmaceutical preparation, and/or cellular therapy and/or gene therapy. For example, engineered T cells may be engineered to express a T cell Receptor (TCRs), or a chimeric antigen receptor (CAR).

Specifically, the host cell is an immune effector cell comprising a chimeric antigen receptor (CAR), such as a CAR-T cell, and the GOI comprises a nucleotide sequence encoding an CAR or part of a CAR, such as any one or more of the CAR domains, that can be expressed by said host cell on its surface.

According to a specific aspect, the host cell is of human origin, yet can be of non-human animal origin, such as of mouse, rat, hamster, dog, pig or cattle.

According to another specific aspect, the host cell is a production host cell as used to produce a protein of interest in an *in vitro* host cell culture.

Specifically, the mammalian cell is a human or rodent or bovine cell, cell line or cell strain. Examples of specific mammalian cells suitable as host cells described herein are mouse myeloma (NSO)-cell lines, Chinese hamster ovary (CHO)-cell lines, HT1080, H9, HepG2, MCF7, MDBK Jurkat, MDCK, NIH3T3, PC12, BHK (baby hamster kidney cell), VERO, SP2/0, YB2/0, Y0, C127, L cell, COS, e.g., COS1 and COS7, QC1-3, HEK-293, VERO, PER.C6, HeLA, EBI, EB2, EB3, oncolytic or hybridoma-cell lines.

The invention further provides for an isolated clone of the host cell described herein, or an *in vitro* cell culture of such clone.

According to a specific aspect, the invention provides for pharmaceutical composition comprising the host cell or a population of host cells described herein, and a pharmaceutically acceptable carrier.

The invention further provides for a preparation comprising a population of host cells, in particular a population or repertoire of different (e.g. polyclonal) cells, wherein at least any one of 1%, 5%, 10%, 15%, 20%, 25%, 30%, 35%, 40%, 45%, 50%, 55%, 60%, 65%, 70%, 75%, 80%, 85%, 90%, or 95%, or 100% of the cells are host cells described herein, with chromosomal identity, or with the identical genotype or identical chromosomotype (i.e. identity based on chromosomal evidence).

Specific examples refer to pharmaceutical preparation comprising a population of CAR-T cells, wherein at least any one of 1%, 5%, 10%, 15%, 20%, 25%, 30%, 35%, 40%, 45%, 50%, 55%, 60%, 65%, 70%, 75%, 80%, 85%, 90%, or 95%, or 100% of the cells are host cells described herein, with chromosomal identity or with the identical genotype or identical chromosomotype, which population stably expresses the GOI, such as to provide for expression over a prolonged period of time.

The invention further provides for an *in vitro* host cell culture of a clone or the preparation described herein, which is maintained under conditions to express said GOI, in particular to stably express said GOI, thereby either obtaining the respective polypeptide or protein of interest e.g. which is bound on the surface of the host cell, or secreted into the host cell culture medium.

The invention further provides for a non-human animal comprising the host cell described herein. Specifically, the animal is a genetically modified animal (also referred to as transgenic animal) comprising a donor sequence comprising the GOI, which donor sequence is inserted at a predetermined insertion site on a chromosome of the animal, wherein the predetermined insertion site is the target site further described herein. An exemplary production method comprises,
a) generating a cell with the donor sequence inserted at the predetermined insertion site; and
b) introducing the cell generated by a) into a carrier animal to produce the genetically modified animal.

Specifically, the cell is a zygote or a pluripotent stem cell.

The invention further provides for a wildcard mammalian host cell comprising heterologous means for site-directed chromosomal integration of an exogenous gene of interest (GOI), in particular a heterologous GOI, at a target site within an intergenic region between a pair of adjacent essential genes. Such wildcard host cell is preferably a diploid host cell.

Exemplary means may be one or more nucleic acid modifications to incorporate a restriction cloning site into the intergenic region, or to incorporate one or more target sites for one or more nucleases. The respective nucleases may be selected from a zinc finger nuclease (ZFN), a TAL-effector domain nuclease (TALEN), or a CRISPR/Cas system. A specific target site may be for a guide RNA (gRNA), such as for a sequence-specific nuclease selected from any of: a TAL-nuclease, a zinc-finger nuclease (ZFN), a meganuclease, a megaTAL, or an RNA guide endonuclease (e.g., Cas9, Cpf1, Cas9 nickase).

Specific means may include one or more nucleic acid modifications to the naturally-occurring intergenic sequence at or adjacent to the target site.

According to a specific aspect, the means are selected from the group consisting of heterologous target sites recognized by a recombinase to mediate the knock-in of a transgene at the respective target site. For example, recognition target sites can be incorporated within the intergenic region as described herein to engineer the target site, and the respective recombinase recognizing such target sites is used to chromosomally integrate the GOI. Any of the commonly used site-directed recombination technology can be used, e.g. those requiring preparatory means to provide the wildcard host cell as described herein. Exemplary site-directed recombination technologies are Flp-FRT recombination, Cre-Lox recombination, or phage lambda site-specific recombination

The invention further provides for a production method to produce recombinant host cells expressing a heterologous GOI, wherein the GOI is inserted at a target site comprised in an intergenic region between a pair of adjacent essential genes.

The invention further provides for a method of producing a gene product encoded by a gene of interest (GOI), by transforming a host cell to chromosomally integrate the GOI within an intergenic region between a pair of adjacent essential genes, and culturing the transformed host cell under conditions to express said GOI.

The invention further provides for a method of modifying a mammalian cell by site directed chromosomal integration of an exogenous, in particular a heterologous GOI, gene of interest (GOI), in particular a heterologous GOI, within an intergenic region between a pair of adjacent essential genes.

According to a specific embodiment, the GOI (or the expression cassette comprising the GOI) is inserted by a method employing a nuclease. The nuclease may comprise a DNA binding domain to bind to the target site, and a nuclease cleavage domain. In particular a nuclease is used to generate a double strand break (DSB) at the target site or within the intergenic region. The double-strand break could facilitate insertion of the transgene by non-homologous end-joining or, in the presence of a donor sequences bearing homologous regions, by homology-directed repair.

Specifically, the method employs any one of a programmable nuclease, such as an engineered endonuclease, preferably a zinc finger nuclease (ZFN), a transcription activator-like effector nuclease (TALEN), a CRISPR endonuclease or an Argonaute protein for chromosomal integration. In particular, the method employs a gene-editing protein selected from a CRISPR/Cas9, TALEN and a zinc finger nuclease.

Specifically, the method employs a gene editing nucleic acid sequence. The gene editing nucleic acid sequence may encode a gene editing molecule selected from the group consisting of: a sequence specific nuclease, one or more guide RNA, CRISPR/Cas, a ribonucleoprotein (RNP), or deactivated CAS for CRISPRi or CRISPRa systems, or any combination thereof. The endonuclease activity of a protein can be assessed by techniques known to those of skill in the art.

Specifically preferred host cells are those transduced with the CRISPR-Cas system.

Argonaute family proteins may as well be used, as well-known in the art. For example, TtAgo, PfAgo and NgAgo have been shown to elicit targeted DNA double-strand breaks that could be used to facilitate transgene insertion by homology-direct repair (Swarts et al. Nature. 2014;507(7491):258-261).

Specifically, the method employs means or method steps of a genome editing technique to insert the GOI at the target site.

According to a specific aspect, the intergenic region is modified to insert, at least one or more of the following:
(i) a gene editing nucleic acid sequence;
(ii) a target site for one or more nucleases;
(iii) a GOI; or
(iv) a guide RNA (gRNA) recognition site for an RNA-guided DNA endonuclease.

According to a specific embodiment, the GOI is inserted into the cellular genome by homologous recombination. For example, a donor sequence comprising the GOI may be inserted into the chromosome at or adjacent to the target site through homologous recombination. Specifically, a vector for gene editing can be used for knock-in of a desired nucleic acid sequence.

To this end, a vector is conveniently used e.g. a viral vector, such as a adeno-associated viral vector, which comprises a target site 5' homology arm, the expression cassette comprising the GOI, and a target site 3' homology arm, wherein the 5' homology arm and the 3' homology arm bind to the target site located in the intergenic region, in particular within the intergenic region, and wherein the 5' and 3' homology arms guide homologous recombination into a locus (in particular a safe harbor locus) located within the intergenic region. Exemplary 5' and 3' homology arms are between 30-2000bp in length. The 5' homology arm and the 3' homology arm particularly bind to target sites that are spatially distinct nucleic acid sequences in the same intergenic region. A certain degree of homology is typically employed e.g. any one or both of the homology arms can be at least any one of 65%, 70%, 75%, 80%, 85%, 90%, 95%, or 98% homology complementary to a target sequence within the intergenic region to hybridize with such complementary target sequence, or be 100% homologous and complementary to the target sequence.

Specific methods of homologous recombination described herein may employ a vector comprising:
a) an expression cassette comprising a promoter and at least one GOI, or comprising a promoter operably linked to at least one GOI; and
b) two self-complementary sequences, e.g., asymmetrical or symmetrical, flanking said expression cassette, which are a 5' homology arm being homologous to a nucleotide sequence upstream of a nuclease cleavage site, and a 3' homology arm being homologous to a nucleotide sequence downstream of the nuclease cleavage site.

The invention further provides for a set of tools for genetically engineering a host cell, comprising
a) a wildcard host cell described herein; and
b) a vector and optionally auxiliary agents adapted to said means for site-directed chromosomal integration of an expression cassette that expresses the GOI upon site directed integration.

Specifically, the GOI is supplied by or comprised in an expression cassette that is incorporated at the target site. The expression cassette may comprise one or more regulatory elements, in particular a promoter, and at least one GOI. Specifically, the expression cassette is a heterologous one.

The invention further provides for a substance, composition of matter or material, which is selected from the host cell, the clone, the preparation, the cell culture, the wildcard host cell, and the set of tools described herein, for medical use, in particular for cellular therapy (e.g. employing CAR-T cells), T cell receptors (for TCR therapy), or cDNAs for gene therapy.

A specific application of the gene therapy involves the treatment of disorders that are either caused by an insufficiency of a secreted gene product or that are treatable by secretion of a therapeutic protein. Such disorders are potentially addressable via delivery of a therapeutic GOI to a number of cells, provided that each recipient cell expresses a high level of the therapeutic GOI. Such applications typically require stable, safe, and high levels of transgene expression.

A therapeutic GOI may be a cDNA which when chromosomally integrated into the target cell is expressed for therapeutic activity, in particular for gene therapy.

Exemplary methods of gene therapy employ a therapeutic GOI expressing a coagulation factor e.g. factor VIII or factor IX, for use in a method of treating hemophilia, such as hemophilia A or B.

Specifically, the substance, composition of matter or material is used as described herein wherein a subject is treated by expressing said GOI *in vivo,* e.g. by a transgenic animal, such as a human being or a non-human animal. Typically, the *in vivo* use is for gene therapy.

The invention further provides for a method of treating a subject comprising administering an effective amount of a substance, composition of matter or material, which is selected from the host cell, the clone, the preparation, the cell culture, the wildcard host cell, and the set of tools described herein, to express said GOI *in vivo.*

According to a specific aspect, the medical use comprises treating a cancer patient by a method comprising:
a) isolating a cell from a mammalian subject, preferably autologous or allogenic to the cancer patient, the cell being an immune cell or hematopoietic cell;
b) modifying the cell by one or more genetic modifications to chromosomally integrate a heterologous gene of interest (GOI) at a target site within an intergenic region between a pair of adjacent essential genes, wherein the GOI encodes a chimeric antigen receptor (CAR); and
c) administering the cell to the subject.

Specifically, the immune cell is a T cell.

The invention further provides for a method for making a chimeric antigen receptor (CAR) T cell, comprising:
(a) obtaining a T cell from a mammalian subject, preferably a human T cell; and
(b) modifying the T cell by one or more genetic modifications to chromosomally integrate a heterologous gene of interest (GOI) at a target site within an intergenic region between a pair of adjacent essential genes, wherein the GOI encodes a chimeric antigen receptor (CAR).

The invention further provides for an *ex vivo* use of the substance, composition of matter or material, which is selected from the host cell, the clone, the preparation, the cell culture, the wildcard host cell, and the set of tools described herein, for *in vitro* engineering transgenic cells or recombinant host cells. Such cells can be provided for expressing the GOI *in vitro* e.g. in a cell culture, or expressing the GOI *in vivo* e.g., upon administering the transgenic cells to a subject in need of such cellular treatment.

### FIGURES

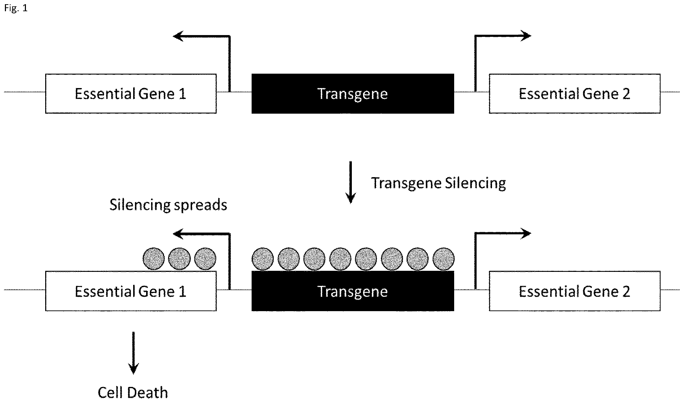

Figure 1: Transgene expression at the indicated locus is not silenced. Transgenes are inserted at a locus that resides between two essential genes, where the essential gene promoters are located in close proximity. Epigenetic silencing of the transgene spreads to one of the neighbouring essential genes (or both). This will lead to cell death. Consequently, all surviving cells maintain transgene expression at high level.

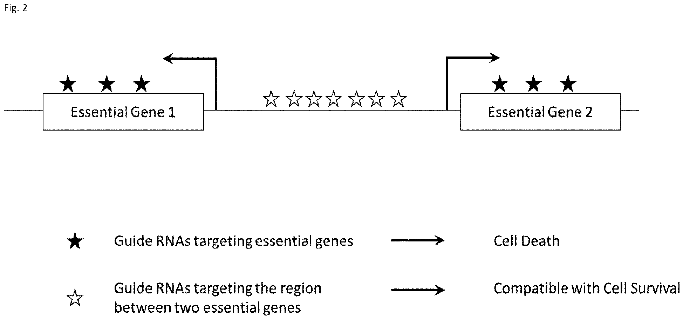

Figure 2: Experimental approach to fine-map the region that allows transgene insertion. Transgene insertion may compromise cell viability. To map the precise location where transgene insertion is tolerated, the region between two essential genes is tiled with an array of guide RNAs. As reference, the neighboring essential genes are also targeted with a suitable set of guide RNAs. Guide RNAs whose activity is tolerated by the cell are identified in the ensuing CRISPR screen.

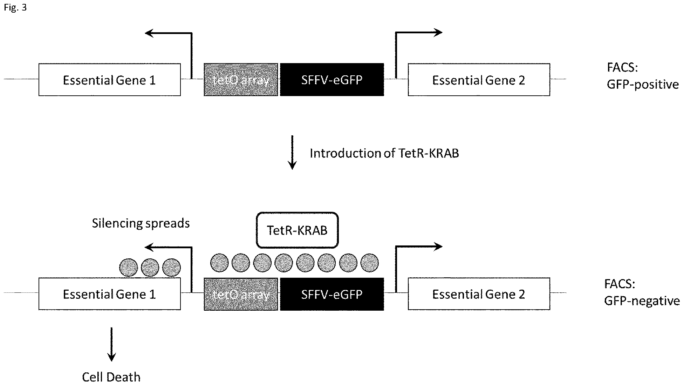

Figure 3: Experimental approach to assess the impact of silencing at the specified locus. A locus between two essential genes is targeted with an expression cassette harboring a reporter gene (eGFP) whose activity is regulated by a suitable constitutive promoter (SFFV). Nearby, an array of TetO sites is included. Upon insertion of the transgene, cells turn GFP-positive as transgene expression is supported. A population of GFP-positive cells is then subjected to treatment with TetR-KRAB. TetR-KRAB silences the TetO sites, which leads to dampened GFP expression. As silencing spreads to the neighbouring essential genes, cells in which GFP silencing has occurred succumb to cell death. GFP-negative survivors are not isolated.

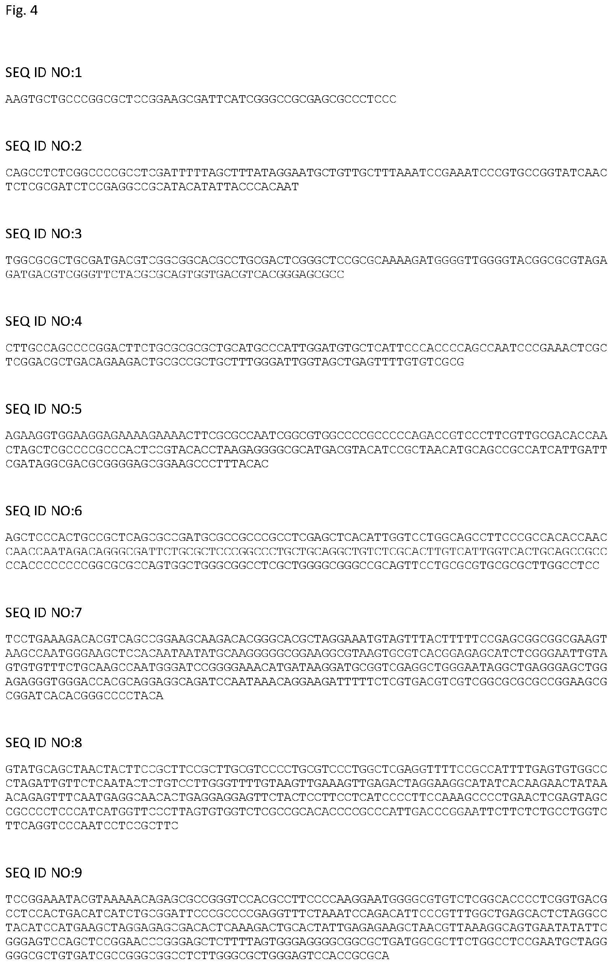

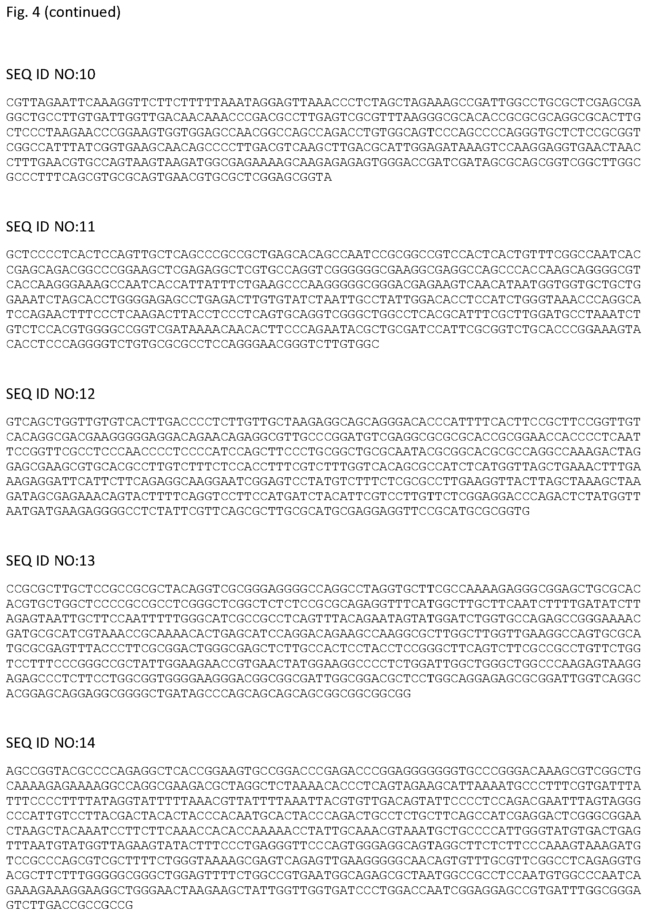

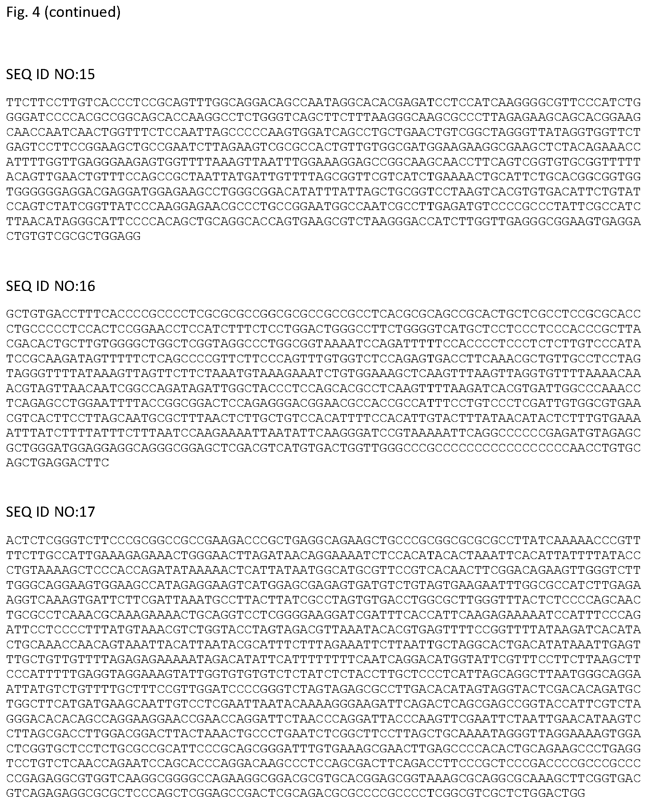

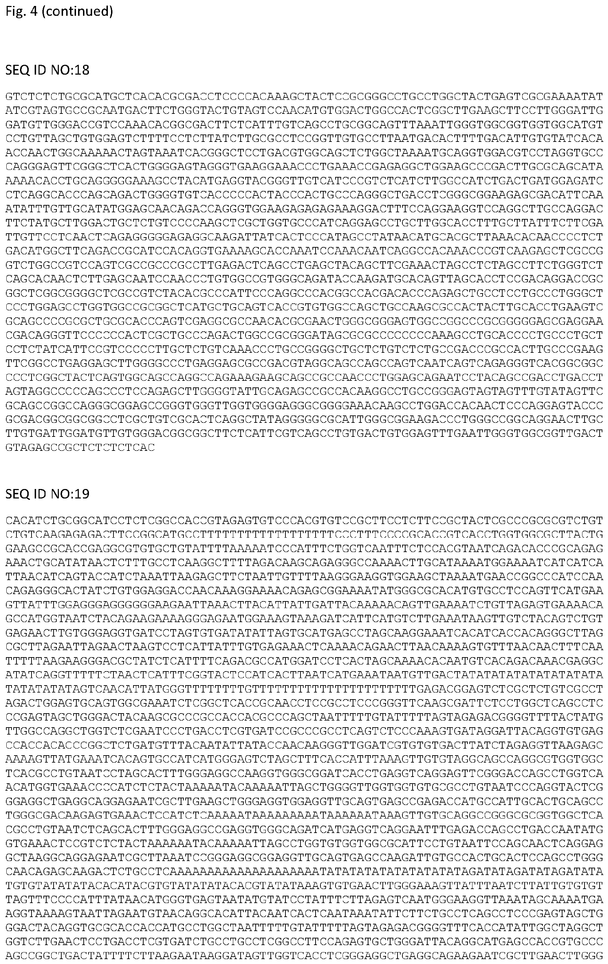

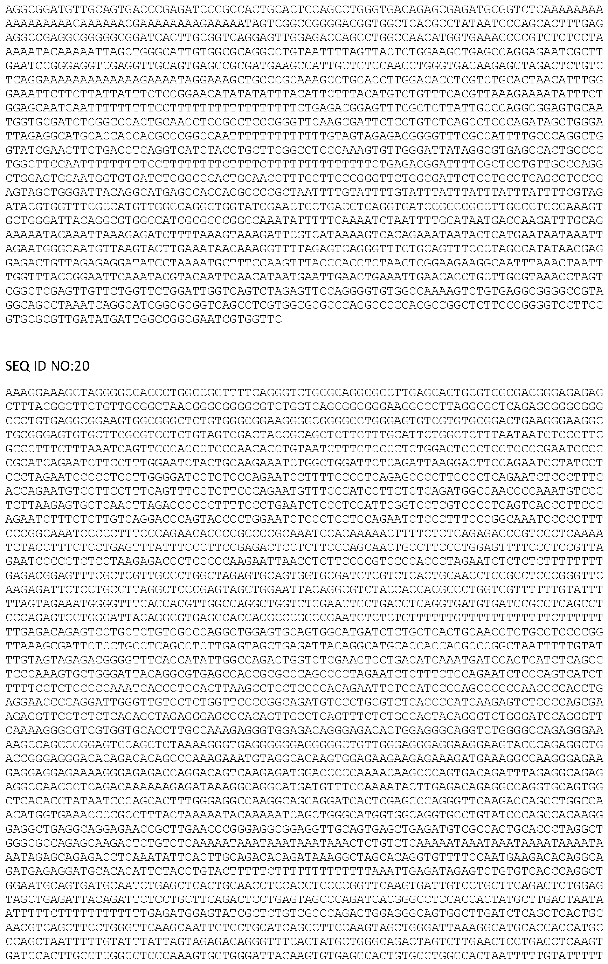

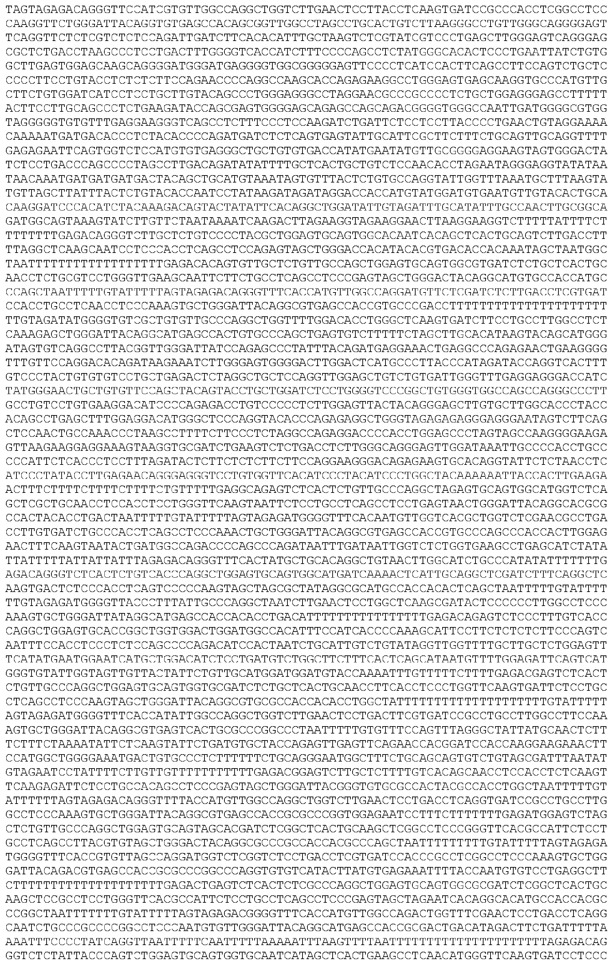

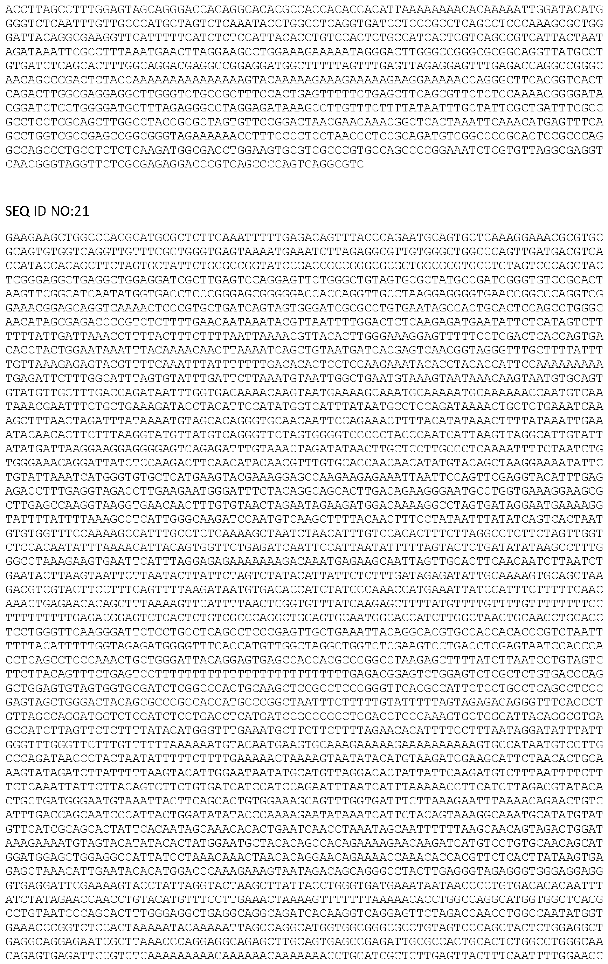

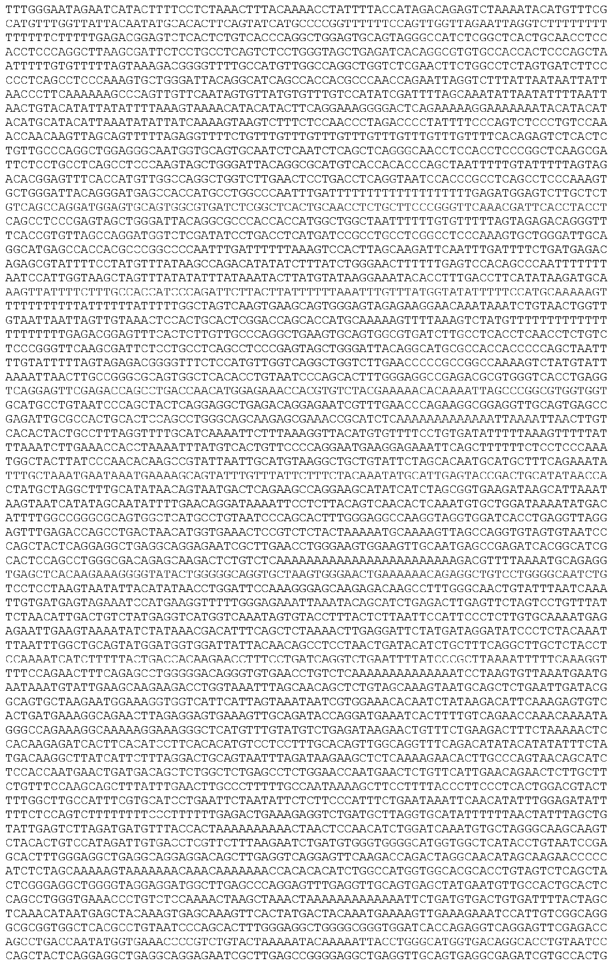

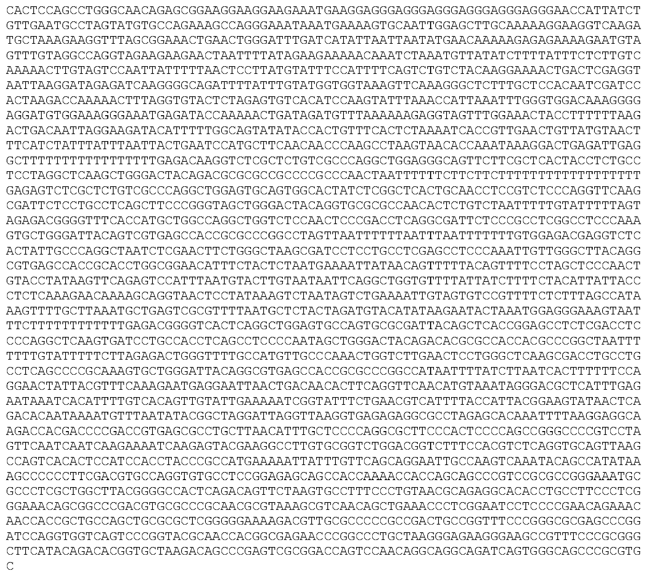

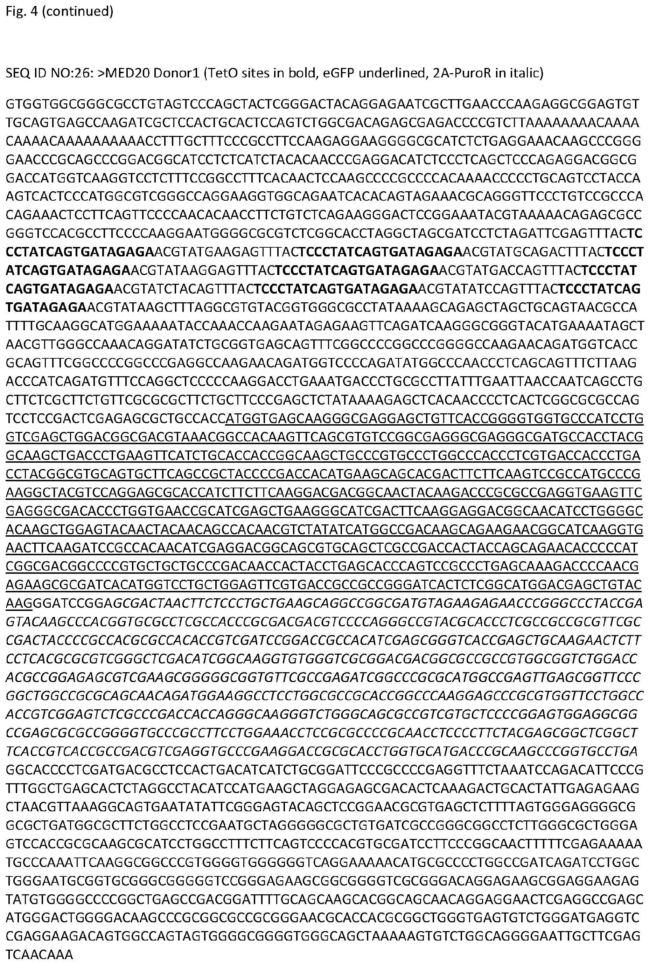

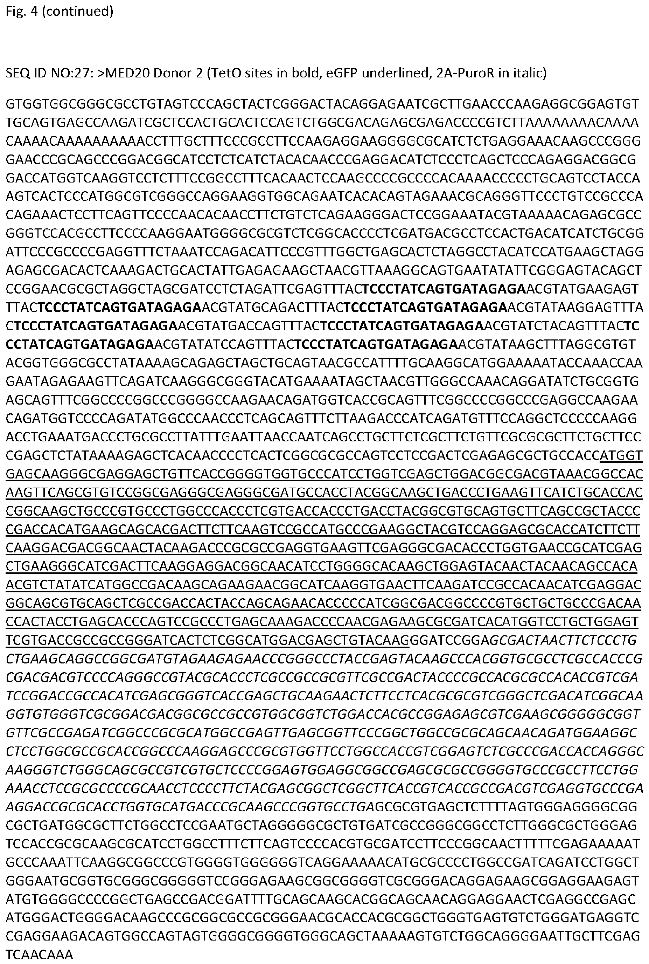

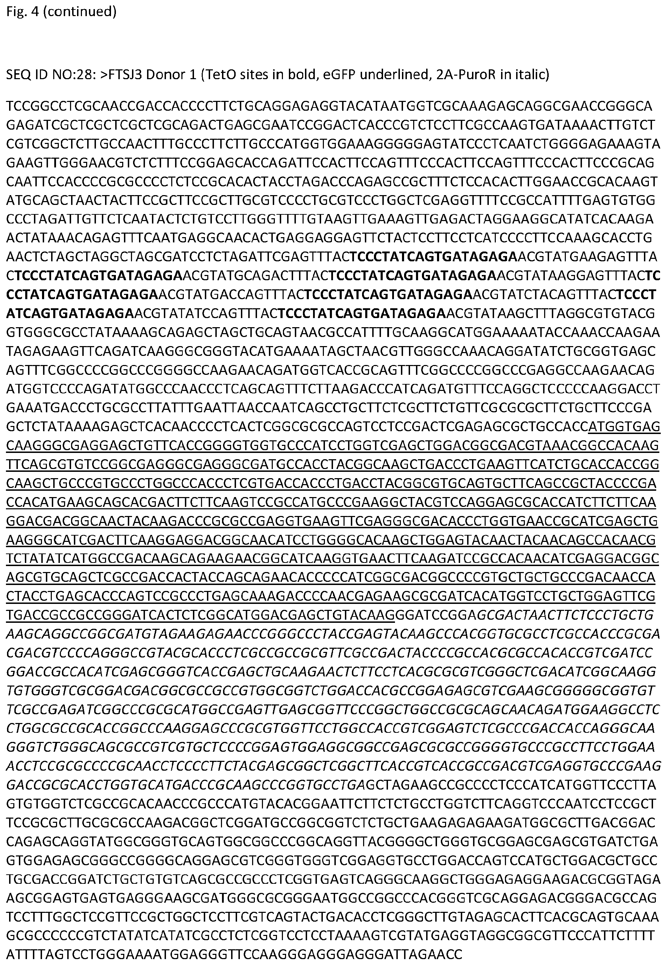

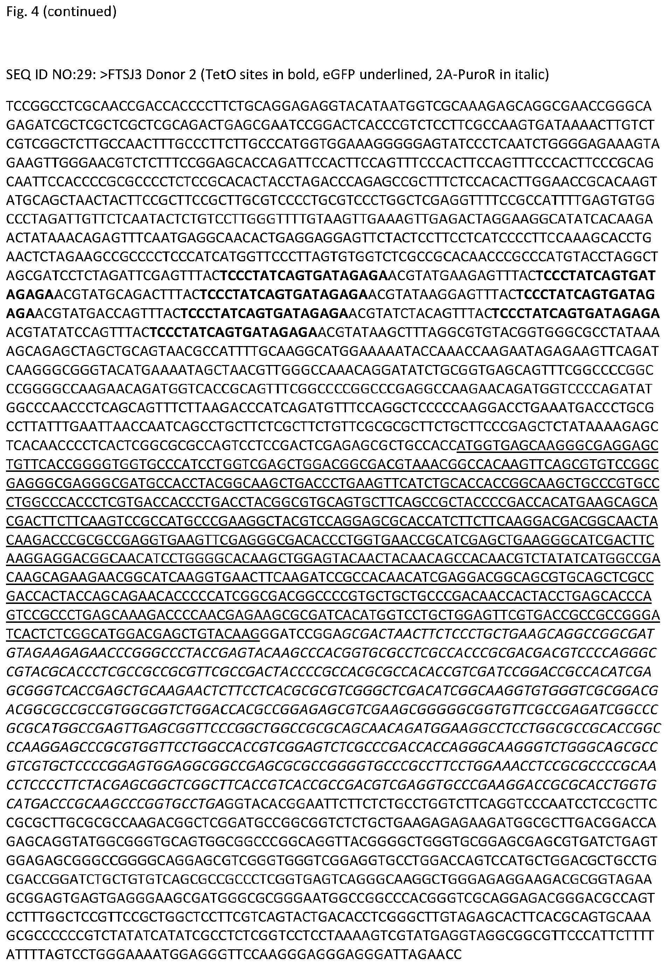

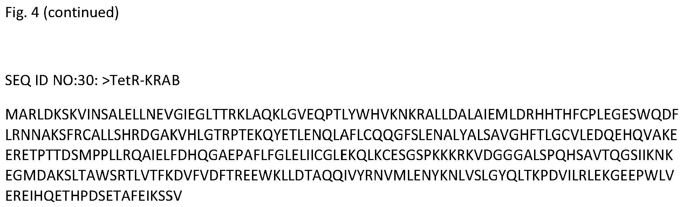

Figure 4: Sequences as herein described.
   SEQ ID NO:1-21: human intergenic regions
   SEQ ID NO:26, >MED20 Donor1 (TetO sites in bold, eGFP underlined, 2A-PuroR in italic).
   SEQ ID NO:27: >MED20 Donor 2 (TetO sites in bold, eGFP underlined, 2A-PuroR in italic).
   SEQ ID NO:28: >FTSJ3 Donor 1 (TetO sites in bold, eGFP underlined, 2A-PuroR in italic).
   SEQ ID NO:29: >FTSJ3 Donor 2 (TetO sites in bold, eGFP underlined, 2A-PuroR in italic).
   SEQ ID NO:30: >TetR-KRAB

### DETAILED DESCRIPTION OF THE INVENTION

Unless indicated or defined otherwise, all terms used herein have their usual meaning in the art, which will be clear to the skilled person. Reference is for example made to the standard handbooks, such as Sambrook et al, "Molecular Cloning: A Laboratory Manual" (2nd Ed.), Vols. 1 -3, Cold Spring Harbor Laboratory Press (1989); Lewin, "Genes IV", Oxford University Press, New York, (1990), and Janeway et al, "Immunobiology" (5th Ed., or more recent editions), Garland Science, New York, 2001.

The subject matter of the claims specifically refers to artificial products or methods employing or producing such artificial products, which may be variants of native (wild-type) products. Though there can be a certain degree of sequence identity to the native structure, it is well understood that the materials, methods and uses of the invention, e.g., specifically referring to isolated nucleic acid sequences, amino acid sequences, fusion constructs, expression constructs, transformed host cells and modified proteins, are "man-made" or synthetic, and are therefore not considered as a result of "laws of nature".

Specific terms as used throughout the specification have the following meaning.

The term "host cell" as used herein is understood as any cell type that is susceptible to transformation, transfection, transduction, or the like with a nucleic acid constructs or expression vectors comprising a polynucleotides encoding expression products described herein, or susceptible to otherwise introduce any or each of the components of the CRISPR complex described herein. The term "host cell" encompasses any progeny of a parent cell that is not identical to the parent cell due to modifications e.g., by a method described herein, or that occur during replication, and shall particularly refer to a single cell, a single cell clone, a population of cells, such as a population comprising polyclonal cells, or a cell line of a host cell.

The term "cell line" as used herein refers to an established clone of a particular cell type that has acquired the ability to proliferate over a prolonged period of time. A cell line is typically used for expressing an endogenous or recombinant gene, or products of a metabolic pathway to produce polypeptides or cell metabolites mediated by such polypeptides. A "production host cell line" or "production cell line", such as used herein for producing a POI, is commonly understood to be a cell line ready-to-use for cell culture in a bioreactor to obtain the product of a production process. A recombinant POI can be produced using the host cell and the respective cell line described herein, by culturing in an appropriate medium, isolating the expressed product or metabolite from the culture, and optionally purifying it by a suitable method.

As used herein, the term "expression" refers to the process by which a polynucleotide is transcribed from a DNA template (such as into and mRNA or other RNA transcript) and/or the process by which a transcribed mRNA is subsequently translated into peptides, polypeptides, or proteins. Transcripts and encoded polypeptides may be collectively referred to as a product of gene expression ("gene product"), such as produced by expressing a GOI. If the polynucleotide is derived from genomic DNA, expression may include splicing of the mRNA in a eukaryotic cell.

The term "expression cassette" as used herein refers to a nucleotide sequence or respective polynucleotide or nucleic acid molecule containing a desired coding sequence and regulatory or control sequences in operable linkage, so that hosts transformed or transfected with these sequences are capable of producing the encoded proteins or host cell metabolites. Exemplary expression control sequences may include any of a promoter, ribosomal binding site, transcriptional or translational start and stop sequences, or of an enhancer or activator sequence.

An expression cassette may comprise at least one intron. Usually, introns are placed at the 5' end of the open reading frame but may also be placed at the 3' end. Said intron may be located between the promoter and or promoter/enhancer element(s) and the 5' end of the open reading frame of the polynucleotide encoding the product of interest to be expressed. Several suitable introns are known in the state of the art that can be used in conjunction with the present disclosure.

In order to effect transformation, the expression system may be included in an expression construct *e*.*g*., in the form of a "vector", or expression cassettes integrated in a host cell's chromosome. Expression cassettes are typically DNA sequences that are required for the transcription of cloned recombinant nucleotide sequences, *i.e.* of recombinant genes and the translation of their mRNA in a suitable host organism. Expression vectors usually comprise one or more of an origin for autonomous replication or a locus for genome integration in the host cells, selectable markers (*e*.*g*., an amino acid synthesis gene or a gene conferring resistance to antibiotics such as zeocin, kanamycin, G418 or hygromycin, nourseothricin), a number of restriction enzyme cleavage sites, and regulatory sequences e.g., any one or more of a suitable promoter sequence, operator, enhancer, ribosomal binding site, and sequences that control transcription and translation initiation and termination. The regulatory sequences are typically operably linked to the DNA sequence to be expressed.

A "vector" is herein understood to be capable of transferring nucleic acid sequences to target cells (e.g., viral vectors, non-viral vectors, particulate carriers, and liposomes). Typically, the vector is understood as an "expression vector" or a "gene transfer vector", which is any nucleic acid construct capable of directing the expression of a nucleic acid of interest and which can transfer nucleic acid sequences to target cells. Thus, the term includes cloning and expression vehicles, as well as viral vectors.

Specific vectors include autonomously replicating nucleotide sequences (e.g. plasmids) as well as genome integrating nucleotide sequences, such as artificial chromosomes e.g., a yeast artificial chromosome (YAC). Expression vectors may include but are not limited to cloning vectors, modified cloning vectors and specifically designed plasmids. Preferred expression vectors described herein are expression vectors suitable for expressing of a recombinant gene in a eukaryotic host cell and are selected depending on the host organism.

The relevant DNA may be integrated into a host cell chromosome. Expression by a host cell may refer to secreted or non-secreted expression products, including polypeptides or metabolites. To allow expression of a recombinant nucleotide sequence in a host cell, the expression cassette or vector described herein typically comprises a promoter operably linked to the GOI, e.g. a promoter nucleotide sequence which is adjacent to the 5' end of the coding sequence, or upstream from and adjacent to a gene of interest (GOI), or if a signal or leader sequence is used, upstream from and adjacent to said signal and leader sequence, respectively, to facilitate expression of the GOI. The promoter sequence is typically regulating and initiating transcription of the downstream nucleotide sequence, with which it is operably linked, including in particular the GOI.

Specifically, the promoter is a heterologous promoter, in particular heterologous to the host cell and/or not natively associated with the GOI.

In specific embodiments, multicloning vectors may be used, which are vectors having a multicloning site. Specifically, a desired heterologous gene can be integrated or incorporated at a multicloning site to prepare an expression vector. In the case of multicloning vectors, a promoter is typically placed upstream of the multicloning site.

Specifically preferred embodiments of a CRISPR system employ a delivery system, comprising one or more vectors, optionally wherein the vectors comprise one or more viral vectors. Specifically, one or more viral vectors may be used which are selected from the group consisting of lentivirus, retrovirus, adenovirus, adeno-associated virus or herpes simplex virus, lentiviral, adenoviral or adeno-associated viral (AAV) vectors. Specific examples are selected from the group consisting of HIV-based lentiviruses. Lentiviral vectors may harbor certain safety features, e.g. they may rely on multiple packaging plasmids or they may have truncated long terminal repeats. All of these features are deemed to reduce the chance of obtaining a replication-competent virus, i.e. typically, these viruses can only undergo a single infection cycle.

In some alternative embodiments, elements of a CRISPR system are delivered via liposomes, particles, cell penetrating peptides, exosomes, microvesicles, or a gene-gun or via electroporation of the target cell.

The expression level can be determined quantitatively or qualitatively, by measuring the mRNA or protein level of said at least one gene, in particular the level of an expression product of a target gene. Specifically, the expression level can be determined by measuring the host cell's transcriptome, or by assessing protein levels at the cell surface by flow cytometry.

In certain embodiments, the expression level is determined using a method selected from the group consisting of quantitative RT-PCR (qPCR), RNA sequencing, hybridization to a microarray, and molecular tagging.

"Gene expression" specifically refers to the conversion of the information, contained in a gene, into a gene product. Gene expression is meant to encompass at least one step selected from the group consisting of DNA transcription into mRNA, mRNA processing, mRNA maturation, mRNA export, translation, protein folding and/or protein transport.

As used herein, the term "gene" e.g. as used in the term "gene of interest" or "GOI" includes a DNA region encoding a gene product, and optionally one or more (or all) DNA regions which regulate the production of the gene product, whether or not such regulatory sequences are adjacent to coding and/or transcribed sequences. Accordingly, a gene may be understood to include promoter sequences, terminators, translational regulatory sequences such as ribosome binding sites and internal ribosome entry sites, enhancers, silencers, insulators, boundary elements, replication origins, matrix attachment sites and locus control regions.

According to further specific examples, a GOI as used in the context of the present invention is a cDNA, and/or encodes a biological product, a ligand (such as an antibody), or a receptor, such as chimeric antigen receptor (CAR) or a T cell receptor.

A suitable CAR may include an antigen-binding part or domain (as an extracellular binding portion), a transmembrane domain, and an intracellular signaling domain, and optionally further includes one or more linker sequences between the various domains.

Specifically, the CAR includes an antigen-binding portion that binds to a target antigen of interest, e.g., a particular antigen on the surface of a target cell. For example, the antigen-binding portion that may be comprised in the CAR may include an antibody, a receptor (e.g., a variable T cell receptor or lymphocyte receptor), a receptor fragment (e.g., an Fc receptor fragment), a ligand, a cytokine, a DARPin, an adnectin, a nanobody, and a peptide.

The antibody included in the antigen-binding portion is preferably specifically recognizing an antigen and may be selected from the group consisting of a full-length antibody, and antibody fragment comprising at least one antibody variable domain or antigen-binding site (preferably a single-chain variable fragment, scFv).

The transmembrane domain specifically fuses the extracellular binding portion and intracellular signaling domain and anchors the CAR to the plasma membrane of the cell.

The intracellular signaling domain of a CAR specifically is the part of a CAR that participates in transducing the signal from CAR binding to a target cell into the interior of the immune effector cell to elicit effector cell function, e.g., activation, cytokine production, proliferation and/or cytotoxic activity, including the release of cytotoxic factors to the CAR-bound target cell, or other cellular responses elicited with target binding to the extracellular CAR domain.

According to a specific aspect, the CAR specifically recognizes and binds to a molecule on the surface of the target cell. According to specific examples, the target cell is a tumor or cancer cell, and the CAR specifically recognizes a tumor associated antigen (TAA), preferably any one of CD19, CD20, CD22, B7-H3 (CD276), CD133, GD2, EGFRvIII, BMSA, MSLN, CEA and HER2.

According to further specific examples, a GOI as used in the context of the present invention is selected from the group consisting of DNA vaccines, or therapeutic DNA molecules e.g., a therapeutic DNA molecule which
i. expresses a functional gene in a subject having a genetic disorder caused by a dysfunctional version of said functional gene (e.g., gene for Duchenne muscular dystrophy, cystic fibrosis, Gaucher's Disease, and adenosine deaminase (ADA) deficiency, inflammatory diseases, autoimmune, chronic and infectious diseases, AIDS, cancer, neurological diseases, cardiovascular disease, hypercholesterolemia, various blood disorders (including various anaemias, thalassemia and haemophilia, and emphysema), and solid tumors);
ii. encodes a toxic peptide (i.e., chemotherapeutic agents such as ricin, diphtheria toxin and cobra venom factor), tumor suppressor genes (such as p53), genes coding for mRNA sequences which are antisense to transforming oncogenes, antineoplastic peptides such as tumor necrosis factor (TNF) and other cytokines, or transdominant negative mutants of transforming oncogenes;
iii. encodes an active RNA form (e.g., a small interfering RNA (siRNA, miRNA, shRNA), or a small activating RNA (saRNA); or,
iv. encodes a CRISPR/Cas component (such as a Cas9 enzyme or an sgRNA).
v. According to further specific examples, the GOI as used in the context of the present invention expresses a protein of interest (POI) e.g. in a cell culture. Specifically, the POI is heterologous to the host cell species.

Specifically, the POI is a secreted peptide, polypeptide, or protein, *i.e.* secreted from the host cell into the cell culture supernatant.

Specifically, the POI is a eukaryotic protein, preferably a mammalian derived or related protein such as a human protein or a protein comprising a human protein sequence, or a bacterial protein or bacterial derived protein

Preferably, the POI is a therapeutic protein functioning in mammals.

In specific cases, the POI is a multimeric protein, specifically a dimer or tetramer.

According to a specific aspect, the POI is a peptide or protein selected from the group consisting of an antigen-binding protein, a therapeutic protein, an enzyme, a peptide, a protein antibiotic, a toxin fusion protein, a carbohydrate - protein conjugate, a structural protein, a regulatory protein, a vaccine antigen, a growth factor, a hormone, a cytokine, a process enzyme, and a metabolic enzyme.

Specifically, the antigen-binding protein is selected from the group consisting of
a) antibodies or antibody fragments, such as any of chimeric antibodies, humanized antibodies, bi-specific antibodies, Fab, Fd, scFv, diabodies, triabodies, Fv tetramers, minibodies, single-domain antibodies like VH, VHH, IgNARs, or V-NAR;
b) antibody mimetics, such as Adnectins, Affibodies, Affilins, Affimers, Affitins, Alphabodies, Anticalins, Avimers, DARPins, Fynomers, Kunitz domain peptides, Monobodies, or NanoCLAMPS; or
c) fusion proteins comprising one or more immunoglobulin-fold domains, antibody domains or antibody mimetics.

A specific POI is an antigen-binding molecule such as an antibody, or a fragment thereof, in particular an antibody fragment comprising an antigen-binding domain. Among specific POIs are antibodies such as monoclonal antibodies (mAbs), immunoglobulin (Ig) or immunoglobulin class G (IgG), heavy-chain antibodies (HcAb's), or fragments thereof such as fragment-antigen binding (Fab), Fd, single-chain variable fragment (scFv), or engineered variants thereof such as for example Fv dimers (diabodies), Fv trimers (triabodies), Fv tetramers, or minibodies and single-domain antibodies like VH, VHH, IgNARs, or V-NAR, or any protein comprising an immunoglobulin-fold domain. Further antigen-binding molecules may be selected from antibody mimetics, or (alternative) scaffold proteins such as *e*.*g*., engineered Kunitz domains, Adnectins, Affibodies, Affiline, Anticalins, or DARPins.

The term "endogenous" as used herein is meant to include those molecules and sequences, in particular endogenous genes or proteins, which are present in a naturally-occurring, wild-type (native) host cell. In particular, an endogenous nucleic acid molecule (e.g., a gene) or protein that does occur in (and can be obtained from) a particular host cell as it is found in nature, is understood to be "host cell endogenous" or "endogenous to the host cell". Moreover, a cell "endogenously expressing" a nucleic acid or protein expresses that nucleic acid or protein as does a host of the same particular type as it is found in nature. Moreover, a host cell "endogenously producing" or that "endogenously produces" a nucleic acid, protein, or other compound produces that nucleic acid, protein, or compound as does a host cell of the same particular type as it is found in nature.

The term "heterologous" as used herein with respect to a nucleotide sequence, construct such as GOI, a promoter, an expression cassette, amino acid sequence or protein, refers to a compound which is either foreign to a given host cell, *i.e.* "exogenous", such as not found in nature in said host cell; or that is naturally found in a given host cell, e.g., is "endogenous", however, in the context of a heterologous construct or integrated in such heterologous construct, *e*.*g*., employing a heterologous nucleic acid fused or in conjunction with an endogenous nucleic acid, thereby rendering the construct heterologous. The heterologous nucleotide sequence as found endogenously may also be produced in an unnatural, *e*.*g*., greater than expected or greater than naturally found, amount in the cell. The heterologous nucleotide sequence, or a nucleic acid comprising the heterologous nucleotide sequence, possibly differs in sequence from the endogenous nucleotide sequence but encodes the same protein as found endogenously. Specifically, heterologous nucleotide sequences are those not found in the same relationship to a host cell in nature. Any recombinant or artificial nucleotide sequence is understood to be heterologous. An example of a heterologous polynucleotide is a nucleotide sequence not natively associated with a promoter, *e*.*g*., to obtain a hybrid promoter, or operably linked to a coding sequence, as described herein. As a result, a hybrid or chimeric polynucleotide may be obtained. A further example of a heterologous compound is a POI encoding polynucleotide operably linked to a transcriptional control element, *e*.*g*., a promoter, to which an endogenous, naturally-occurring POI coding sequence is not normally operably linked.

The term "isolated" or "isolation" as used herein shall refer to such compound that has been sufficiently separated from the environment with which it would naturally be associated, so as to exist in "purified" or "substantially pure" form. The term "isolated" can refer to material that is free, substantially free, or essentially free to varying degrees from components which normally accompany it as found in its native state. "Isolate" also denotes a degree of separation from original source or surroundings. Yet, "isolated" does not necessarily mean the exclusion of artificial or synthetic mixtures with other compounds or materials, or the presence of impurities that do not interfere with the fundamental activity, and that may be present, for example, due to incomplete purification. Isolated compounds can be further formulated to produce preparations thereof, and still for practical purposes be isolated - for example, host cells or a POI can be mixed with pharmaceutically acceptable carriers or excipients when used in diagnosis or therapy.

As used herein, the term "isolated cell" refers to a cell that is separated from the molecular and/or cellular components that naturally accompany the cell.

The term "operably linked" as used herein refers to the association of nucleotide sequences on a single nucleic acid molecule, *e*.*g*., a vector, or an expression cassette, in a way such that the function of one or more nucleotide sequences is affected by at least one other nucleotide sequence present on said nucleic acid molecule. By operably linking, a nucleic acid sequence is placed into a functional relationship with another nucleic acid sequence on the same nucleic acid molecule. For example, a promoter is operably linked with a coding sequence of a recombinant gene, when it is capable of effecting the expression of that coding sequence. As a further example, a nucleic acid encoding a signal peptide is operably linked to a GOI or a nucleic acid sequence encoding a POI, when it is capable of expressing a protein in the secreted form, such as a preform of a mature protein or the mature protein. Specifically, such nucleic acids operably linked to each other may be immediately linked, *i.e.* without further elements or nucleic acid sequences in between the nucleic acid encoding the signal peptide and the nucleic acid sequence encoding a POI.

The term "recombinant" as used herein shall mean "being prepared by or the result of genetic engineering. A recombinant host may be engineered to insert one or more nucleotides, polynucleotides or nucleotide sequences, and may specifically comprise an expression vector or cloning vector containing a recombinant nucleic acid sequence, in particular employing nucleotide sequence foreign to the host. A recombinant host cell can be produced by using genetic engineering, *i.e.* by human intervention, such as to insert a GOI at a certain chromosomal locus. When a host cell is engineered to incorporate a GOI at a safe harbor locus for stable expression of the GOI, the host cell is manipulated such that the host cell has the capability to express such gene at a certain level over a prolonged period of cultivation (e.g., *in situ, in vivo, ex vivo* or *in vitro*) which is higher than the expression level of the host cell under the same condition prior to manipulation, or compared to the host cells which are not engineered for GOI expression.

Genetic engineering is conveniently performed by genome editing techniques, homologous recombination or other site-directed recombination technologies such as by Flp-FRT recombination, Cre-Lox recombination, or phage lambda site-specific recombination

Flp-FRT recombination is understood as a site-directed recombination technology and involves the recombination of sequences between short flippase recognition target (FRT) sites by the recombinase flippase (Flp) derived from the 2 µ plasmid of baker's yeast Saccharomyces cerevisiae.

Cre-Lox recombination is understood as a site-specific recombinase technology. The system consists of a single enzyme, Cre recombinase, which recombines a pair of short target sequences called the *Lox* sequences.

Phage lambda site-specific recombination employs topoisomerase activity of bacteriophage lambda Int protein introducing single-strand breaks into duplex DNA at recognition sequences.

The term "recombinant" with respect to a cell, GOI or POI as used herein, includes a cell, molecule or product of interest that is prepared, expressed, created or isolated by recombinant means, such as a cell engineered to express a GOI as described herein, a GOI chromosomally integrated into a host cell as described herein, or a POI produced by a host cell transformed to express the POI, as described herein. In accordance with the present invention conventional molecular biology, microbiology, and recombinant DNA techniques within the skill of the art may be employed. Such techniques are explained fully in the literature. See, *e*.*g*., Maniatis, Fritsch & Sambrook, "Molecular Cloning: A Laboratory Manual, Cold Spring Harbor, (1982).

The term "sequence identity" of a variant, homologue or orthologue as compared to a parent nucleotide or amino acid sequence indicates the degree of identity of two or more sequences. Two or more amino acid sequences may have the same or conserved amino acid residues at a corresponding position, to a certain degree, up to 100%. Two or more nucleotide sequences may have the same or conserved base pairs at a corresponding position, to a certain degree, up to 100%.

Sequence similarity searching is an effective and reliable strategy for identifying homologs with excess (*e*.*g*., at least 50%) sequence identity. Sequence similarity search tools frequently used are *e*.*g*., BLAST, FASTA, and HMMER.

Sequence similarity searches can identify such homologous proteins or genes by detecting excess similarity, and statistically significant similarity that reflects common ancestry. Homologues may encompass orthologues, which are herein understood as the same protein in different organisms, *e*.*g*., variants of such protein in different different organisms or species.

To determine the % complementarity of two complementary sequences, one of the two sequences needs to be converted to its complementary sequence before the % complementarity can then be calculated as the % identity between the first sequence and the second converted sequences using the above-mentioned algorithm.

"Percent (%) amino acid sequence identity" with respect to an amino acid sequence, homologs and orthologues described herein is defined as the percentage of amino acid residues in a candidate sequence that are identical with the amino acid residues in the specific polypeptide sequence, after aligning the sequence and introducing gaps, if necessary, to achieve the maximum percent sequence identity, and not considering any conservative substitutions as part of the sequence identity. Those skilled in the art can determine appropriate parameters for measuring alignment, including any algorithms needed to achieve maximal alignment over the full length of the sequences being compared.

For purposes described herein, the sequence identity between two amino acid sequences is determined using the NCBI BLAST program version BLASTP 2.8.1 with the following exemplary parameters: Program: blastp, Word size: 6, Expect value: 10, Hitlist size: 100, Gapcosts: 11.1, Matrix: BLOSUM62, Filter string: F, Compositional adjustment: Conditional compositional score matrix adjustment.

"Percent (%) identity" with respect to a nucleotide sequence *e*.*g*., of a nucleic acid molecule or a part thereof, in particular a coding DNA sequence, is defined as the percentage of nucleotides in a candidate DNA sequence that is identical with the nucleotides in the DNA sequence, after aligning the sequence and introducing gaps, if necessary, to achieve the maximum percent sequence identity, and not considering any conservative substitutions as part of the sequence identity. Alignment for purposes of determining percent nucleotide sequence identity can be achieved in various ways that are within the skill in the art, for instance, using publicly available computer software. Those skilled in the art can determine appropriate parameters for measuring alignment, including any algorithms needed to achieve maximal alignment over the full length of the sequences being compared.

Optimal alignment may be determined with the use of any suitable algorithm tor aligning sequences, non-limiting examples of which include the Smith-Waterman algorithm, the Needleman-Wunsch algorithm, algorithms based on the Burrows-Wheeler Transform (e.g., the Burrows Wheeler Aligner), ClustalW, Clustal X, BLAT, Novoalign (Novocraft Technologies; available at novocraft.com), ELAND (Illumina, San Diego, CA), SOAP (available at soap.genomies.org.cn), and Maq (available at maq.sourceforge.net).

The term "stable" as used herein in the context of expression, expressers or expression constructs, means that a host cell is capable of correctly expressing the GOI over a prolonged period of time. A stable expresser is specifically understood to refer to a host cell maintaining the genetic properties, specifically keeping a GOI expression at a high and/or about constant level, even after about 20 generations, preferably at least 30 generations, more preferably at least 40 generations, most preferred of at least 50 generations. Specific embodiments refer to a stable expression during at least 5, or 10, or 20, or 30, or 40, or 50 doublings or passages.

According to a specific aspect, a population of host cells is provided wherein said cells comprise stably integrated into their chromosome a heterologous GOI, wherein on average at least 20%, at least 30%, at least 40%, at least 50%, at least 60% of the cells originating from said population do not lose more than 70%, preferably not more than 50%, of their gene product expression titer over a time period of at least 4 weeks, preferably 8 weeks, preferably 10 weeks, more preferably over a time period of 12 weeks. Expression may be monitored ex-vivo (in cell culture) or in vivo (upon transplantation). As can be shown in the examples, after transfection and identification of stably transfected cells, the amount of cells which do not show a gradual loss in productivity during prolonged culturing is increased when using the cells described herein, i.e. more stable cell clones are obtained from a selected cell population. The stability property can be tested, by cultivating individual cells from said population as cell clones and determining the titer over the indicated time period. The host cell's expression of the GOI can be determined by various methods, e.g. by ELISA, by Western blotting, by radioimmunoassays, by immunoprecipitation, by assaying for the biological activity of the gene product, or by immunostaining followed by FACS analysis. In a specific aspect, the expression of the GOI is determined by Western blotting.

The stability can be tested by the absence of gene silencing, such as described in Example 3. It can also be tested by culturing cells over extended period of time and monitoring transgene expression levels in vitro or in vivo, by methods known in the art. As transgene insertion may occur in vivo, transgene expression can be monitored in vivo, e.g. by taking patient samples (e.g. blood or biopsies from the relevant tissue). Typically, the expression is considered stable, if transgene expression is about the same (+/-50% or +/-40% or +/- 30% or +/- 20% or +/- 10%) during the observation period.

The stability rates can vary from project to project depending on the expressed gene product. However, the abundance of cells with stable expression characteristics can be significantly increased in the population of successfully transduced host cells. Therefore, the risk that an instable clone which gradually loses productivity during prolonged culturing is chosen for therapy or any other industrial application can be significantly reduced.

Specifically, a stable recombinant host cell line is provided which is considered a great advantage when used for industrial scale production.

Stable expression can be effected upon transduction, e.g. by genome editing or other approaches for targeted transgene insertion known in the art, thereby integrating an expression construct into the host cell's genome, wherein the targeted insertion is within a predetermined chromosomal region that is considered to be a "safe harbor", such as within an intergenic region as further described herein. Stable transduction is preferred over a transient one to generate high expressing host cells or clones for expressing a GOI and producing a gene product, or a POI *in vivo,* or *in vitro* such as on industrial scale. A population of stable expressers includes a relative high proportion of high and stable producing clones. Stability is particularly increased when incorporating a GOI into a safe harbor locus such as within the intergenic region as described herein, thereby reducing or avoiding transcriptional gene silencing.

In the context of the present invention, the stable expression of a heterologous GOI is achieved by allowing the respective expression construct to be inserted into the intergenic region between two adjacent essential genes, as described herein. Such intergenic region is understood as a hot spot region, meaning that it supports high transcription of introduced genes and that this transcription is stable over time and reproducible for different genes and different culture conditions.

The term "essential gene" is herein understood to include those genes of a host cell coding for an essential polypeptide, and is preferably a gene that has not been shown to be non-essential in the host cell. In one embodiment, the essential gene is a gene whose deficiency renders the host cell non-viable under certain culture conditions because of lower or no expression in the host cell of the essential polypeptide. The essential polypeptide may be a polypeptide able to produce a nutrient which is essential for cell viability (e.g. wherein the essential polypeptide is an enzyme able to produce the nutrient essential for cell viability) or a polypeptide involved in the production of a nutrient which is essential for cell viability (e.g. wherein the essential polypeptide is an enzyme involved in the metabolic pathway which leads to the production of a nutrient essential for cell viability). Yet, the essential gene coding for the essential polypeptide may be a gene whose deficiency renders the host cell non-viable under all conditions and in any type of nutrient medium. The essential polypeptide may be a polypeptide whose lower or no expression in the host cell renders the host cell non-viable under all conditions and in any nutrient medium, such as minimal or complex medium. Preferably, the essential gene coding for an essential polypeptide is a gene essential in eukaryotes coding for a polypeptide essential in eukaryotes. Suitable examples of classes of essential genes include, but are not limited to, genes involved in DNA synthesis and modification, RNA synthesis and modification, protein synthesis and modification, proteasome function, the secretory pathway, cell wall biogenesis and cell division. It is well-known that many genes encoding compounds involved in primary metabolism and metabolic pathways can be essential genes, whose deficiency renders the host cell non-viable.

The term "wildcard host cell" shall mean a host cell, which is prepared by genetic engineering to comprise an artificial or heterologous nucleotide sequence, such as described herein for site-directed insertion of a GOI, and which is ready to incorporate the GOI. A wildcard cell is also understood as an "empty" host cell (i.e. a recombinant host cell without the transgene) that can be used e.g. as cloning cell line for recombinant production technologies. A respective cell can be transfected with a heterologous GOI, e.g. using an appropriate expression vector.

The wildcard cell line is thus a recombinant host cell line, which is characterized for its expression capacity of any desired GOI. This follows an innovative "wildcard" strategy for the generation of producer cell lines, e.g. using site-specific recombinase-mediated cassette exchange or homologous recombination. Such a new host cell facilitates the cloning of a GOI, e.g. into predetermined genomic expression hot spots within days in order to get reproducible, highly efficient production cell lines.

Therefore, the present invention provides for a novel solution to overcome transgene silencing by selecting safe harbour loci in the genome that cannot be silenced. Silencing is a phenomenon that is implemented by establishing repressive marks on DNA or histones (e.g. trimethylation of histone H3 on lysine 9 or 27). Herein described are target regions, each being surrounded by two essential genes. Preferably, the two essential genes are oriented such that their promoters are immediately adjacent to the safe harbour site and face in opposite directions. And preferably, the two promoters are very close to one another, thus ensuring that silencing of the transgene, if it occurred, would silence the neighbouring essential genes. Consequently, silencing of the transgene would lead to cell death, thus implementing a mechanism by which one can select for cell types and cell states in which no silencing of the transgene occurs.

The essential genes are preferably highly essential across multiple cell types and thus belong to the "core essential gene set", pertaining to genes (i) which are highly essential and thus absolutely required and (ii) which are essential in every cell of a higher organism, such as the human body, in order to make the strategy universally applicable. Suitable pairs of essential genes are ideally close to one another (preferably with their promoter regions), such as to allow the introduction of a transgene in between them. An exemplary selection of around 20 suitable loci, flanked by two highly essential genes is particularly provided.

A transgene can be inserted into the described safe harbour loci in several ways. Those include the use of Zinc finger nucleases in which an array of Zinc finger proteins mediates binding to a specific genomic sequence and Fokl nuclease triggers the DNA double-strand break near the Zinc finger binding site. Alternatively, Transcription Activator-like Effector Nuclease (TALENs) consisting of an array of plant-derived transcription activator-like effectors and Fokl nuclease is equally suited. In addition, DNA- or RNA-guided nucleases can be used. Those include the popular CRISPR enzymes Cas9, Cas12a/ Cpf1 or Cas12b, CasX, but also more complex CRISPR systems such as the Cascade complex. It may also include bacterial Argonautes which have been used for targeted DNA double-strand break induction. The transgene can also be delivered by a combination of a programmable endonuclease with AAV-derived DNA donor, which is often chosen to enhance rates of homologous recombination.

The expression of transgenes from safe harbour loci is a particularly relevant when creating human or other (e.g. CHO) cell lines expressing high levels of gene products, such as antibodies or cytokines, utilized as production cell lines.

It can also be exploited for gene and cell therapy. For instance, it can be used to introduce a chimeric antigen receptor (CAR) into a T cell in a targeted fashion in order to ensure that the CAR is stably expressed and is not silenced during T cell expansion or re-perfusion. Likewise, it can be applied in T cell receptor (TCR) therapy where the endogenous TCR is replaced with an exogenous TCR in order to reprogram the specificity of a T cell and engineer it to recognize a novel target cell (e.g. a tumour cell).

The approach described here can also be utilized to engineer stable induced pluripotent stem (iPS) cell lines which express a transgene to high levels and where transgene expression is unaffected by iPS cell differentiation. The latter remains a significant problem in stem cell biology.

Transgene expression can be monitored at the mRNA or protein level in various ways. mRNA levels can be quantified by quantitative RT-PCR or by RNA sequencing. Protein levels can be determined by Western Blot or ELISA. At the single cell level, protein expression can be determined by flow cytometry. This does not only report the protein level in individual cells, but also reports the fraction of cells in a population that express the transgene. When a transgene is well expressed, its expression can be monitored in single cells by flow cytometry, thus reporting which fraction of a total population expresses the transgene. At the DNA level, the fraction of cells harbouring the transgene can be estimated by digital droplet PCR. Alternatively, one may sort single cells into individual wells of a 96well plate and determine the fraction of transgene-positive cells by a PCR-based approach.

The issue of gene silencing can be addressed in two ways:
Human iPS cells can be utilized and an expression cassette introduced into the candidate safe harbour locus using CRISPR/Cas9. The expression cassette contains a strong promoter (e.g. EF1A) driving eGFP or mCherry, thus ensuring that the transgene is strongly expressed. Once a stable clone has been isolated in which transgene integration has been verified by PCR, iPS cells are differentiated into various lineages (e.g. neurons or cardiomyocytes). Transgene expression is monitored by FACS throughout the differentiation process to ensure that expression is stably maintained and not subject to silencing.

A human cell line (e.g. HEK293, Jurkat, U937, Hela or human iPS cells) is used to introduce an expression cassette into the candidate safe harbour locus using CRISPR/Cas9. The expression cassette contains eGFP driven by a promoter (e.g. SFFV, CMV or EF1As) that is flanked by Tetracycline resistance (tetO) sequences. In the absence of TetR-KRAB, eGFP is strongly expressed in the recipient cells and a stable population or a clonal cell line can be obtained by FACS sorting. Introduction of TetR-KRAB leads to the silencing of eGFP. However, the silencing will spread to the neighbouring promoters governing two essential genes which leads to the death of the cells in which effective silencing occurs. Consequently, one would expect that all cells that lose eGFP expression will die. Survival of a significant fraction of eGFP-negative cells indicates that, contrary to such expectation, silencing of this locus can indeed occur.

The foregoing description will be more fully understood with reference to the following examples. Such examples are, however, merely representative of methods of practicing one or more embodiments of the present invention and should not be read as limiting the scope of invention.

### EXAMPLES

### Example 1: CRISPR screen to fine map the dual essential gene loci

Transgene insertion between two essential genes could be harmful to the recipient cell as it may alter the expression of at least one of the two essential genes. Hence, it is assessed which guide RNAs can be utilized for transgene insertion without compromising cell fitness. To identify these, the entire region between the transcriptional start sites of each essential gene pair is tiled with all possible guide RNAs (Figure 2) and their fitness phenotype is assessed in a dropout screen. As references, guide RNAs targeting the coding regions of the essential genes in question are included, thus confirming that these genes are indeed essential. Negative controls are also included, i.e. guide RNAs targeting non-essential genes or intergenic regions.

### Example 2: Targeting safe harbour loci and addressing the "silenceability" experimentally

Two human gene pairs have been identified and targeting the respective intergenic regions is evaluated. The two gene pairs are: MED20/ BYSL and FTSJ3/ PSMC5.

The first pair of essential genes: the first gene is FTSJ3 (GeneID: 117246) and the second gene is PSMC5 (GeneID: 5705), intergenic region: SEQ ID NO:8.

The second pair of essential genes; the first gene is MED20 (GeneID: 9477) and the second gene is BYSL (GeneID: 705), intergenic region: SEQ ID NO:9.

For each of them, the region between the two essential genes is targeted using the following guide RNAs:

MED20 guide RNA 1 (SEQ ID NO:22):
GGGCGUGUCUCGGCACCCCUGUUUCAGAGCUAUGCUGGAAACAGCAUAGCAAGUUGAAAUAA
GGCUAGUCCGUUAUCAACUUGAAAAAGUGGCACCGAGUCGGUGCUUUUU

MED20 guide RNA 2 (SEQ ID NO:23):
GAGCUCCCGGGUUCCGGAGCGUUUCAGAGCUAUGCUGGAAACAGCAUAGCAAGUUGAAAUAA
GGCUAGUCCGUUAUCAACUUGAAAAAGUGGCACCGAGUCGGUGCUUUUU

FTSJ3 guide RNA 1 (SEQ ID NO:24):
GGGGCGGCUACUCGAGUUCAGUUUCAGAGCUAUGCUGGAAACAGCAUAGCAAGUUGAAAUAA
GGCUAGUCCGUUAUCAACUUGAAAAAGUGGCACCGAGUCGGUGCUUUUU

FTSJ3 guide RNA 2 (SEQ ID NO:25):
GAAUUCCGGGUCAAUGGGCGGUUUCAGAGCUAUGCUGGAAACAGCAUAGCAAGUUGAAAUAAG
GCUAGUCCGUUAUCAACUUGAAAAAGUGGCACCGAGUCGGUGCUUUUU

An expression cassette (Figure 3) is introduced into each of the specified loci by homology-directed repair. The expression cassette harbours eGFP linked to Puromycin resistance via a 2A peptide, the expression of which is driven by an SFFV promoter. The SFFV promoter is flanked by an array of seven tetO sites as specified in the respective donor sequences, Figure 4:
SEQ ID NO:26, >MED20 Donor1 (TetO sites in bold, eGFP underlined, 2A-PuroR in italic).
SEQ ID NO:27: >MED20 Donor 2 (TetO sites in bold, eGFP underlined, 2A-PuroR in italic).
SEQ ID NO:28: >FTSJ3 Donor 1 (TetO sites in bold, eGFP underlined, 2A-PuroR in italic).
SEQ ID NO:29: >FTSJ3 Donor 2 (TetO sites in bold, eGFP underlined, 2A-PuroR in italic).

HEK293 cells are transduced with a Cas9 expression plasmid, alongside with the following combination of guide RNA expression plasmid and donor plasmid:
MED20 guide RNA 1 with MED20 donor 1
MED20 guide RNA 2 with MED20 donor 2
FTSJ3 guide RNA 1 with FTSJ3 donor 1
FTSJ3 guide RNA 2 with FTSJ3 donor 2

Following expression of Cas9 and the guide RNA, the donor is incorporated by homology-directed repair at the target site. Cells bearing the cognate insertion of the transgene cassette can be selected for using puromycin and enriched by subsequent FACS sorting. Ultimately, clones bearing the transgene insertion are obtained by limiting dilution and will be genotyped using PCR.

Once a stable cell line (monoclonal or polyclonal) has been obtained, cells are transfected with an expression cassette harbouring the TetR-KRAB construct specified in Figure 4, SEQ ID NO:30.

The TetR-KRAB construct, upon introduction in the recipient cell, is expressed and binds to the array of tetO sites. Binding of TetR-KRAB induces silencing of the SFFV promoter, thus effectively silencing the eGFP transgene. However, silencing is spread to the adjacent promoters governing the two essential genes (MED20/ BYSL or FTSJ3/ PSMC5). Consequently, cells in which eGFP silencing has occurred die.

Surviving cells are assayed by flow cytometry for GFP positivity. To prove that the novel safe harbour loci that are located between two essential genes are functional, there are no surviving cells that are GFP-negative and all surviving cells maintain GFP expression.

### Example 3 Assessing transgene silencing in human iPS cells

Human iPS cell lines such as BOB-C or KOLF2 are used, which are obtained from the HipSci consortium. A GFP reporter under the control of a constitutive promoter (e.g. CMV, SFFV, EF1As, EF1A) is introduced into the safe harbour locus of interest. Two exemplary combinations of guide RNA and donor vector are provided in Example 2. In contrast to Example 2, the donor vector does not harbour any TetO sites.

Following introduction via CRISPR-Cas9 as specified in Example 2, a stable population of cells (polyclonal or monoclonal) harbouring the transgene at the desired locus is obtained. This population is differentiated into various lineages using protocols known in the art (Volpato V et al. Stem Cell Reports. 2018; 11(4):897-911; Mummery CL et al. Circ Res. 2012;111(3):344-358). Transgene expression is assessed prior to differentiation and at the various stages of differentiation (early, late, terminal). Depending on the target cell type, differentiation takes from 15 days to 120 days, and differentiation is monitored using suitable markers known in the art. To prove that no silencing occurs, transgene expression is stably detected (e.g. by flow cytometry) during the entire course of the experiment. Clones in which silencing of the transgene occurred succumb to cell death as the silencing inevitably spreads to at least one of the neighbouring essential genes (Figure 1).

## Claims

1. An isolated mammalian host cell comprising a heterologous gene of interest (GOI) chromosomally integrated at a target site within an intergenic region between a pair of adjacent essential genes.

2. The host cell of claim 1, wherein the length of the intergenic region is less than 20.000 nt.

3. The host cell of claim 1 or 2, wherein the intergenic region comprises or consists of the nucleic acid sequence which is at least 90% identical to any one of SEQ ID NO:1-21.

4. The host cell of any one of claims 1 to 3, wherein the essential genes of said pair are oriented such that their promoters are adjacent and face in opposite directions.

5. The host cell of any one of claims 1 to 4, wherein the GOI is comprised in a heterologous expression cassette integrated at the target site.

6. The host cell of any one of claims 1 to 5, which is of a cell type selected from the group consisting of a Natural Killer (NK) cell, or a a T cell, such as a cytotoxic T lymphocyte (CTL), a regulatory T cell, or a T helper cell.

7. The cell of claim 6, which is a CAR-T cell, wherein the GOI encodes a chimeric antigen receptor expressed by said CAR-T cell.

8. A preparation comprising a population of mammalian host cells, wherein at least 1% of the cells are host cells of any one of claims 1 to 7 with chromosomal identity.

9. An *in vitro* host cell culture of a host cell of any one of claims 1 to 7, which is maintained under conditions to express said GOI.

10. A method of producing a gene product encoded by a gene of interest (GOI), by transforming a host cell to chromosomally integrate the GOI within an intergenic region between a pair of adjacent essential genes, and culturing the transformed host cell under conditions to express said GOI.

11. A method of modifying a mammalian cell by site directed chromosomal integration of a heterologous gene of interest (GOI) within an intergenic region between a pair of adjacent essential genes.

12. The method of claim 10 or 11, which employs any one of a programmable nuclease, preferably a zinc finger nuclease (ZFN), a transcription activator-like effector nuclease (TALEN), a CRISPR endonuclease or an Argonaute protein for chromosomal integration.

13. A host cell of any one of claims 1 to 7, or the preparation of claim 8, for medical use.

14. The host cell of any one of claims 1 to 7, or the preparation of claim 8, for use according to claim 13, wherein a subject is treated by expressing said GOI *in vivo.*

15. An *ex vivo* use of the host cell of any one of claims 1 to 7 in a method of producing induced pluripotent stem cells, antibody production, and/or production of a protein of interest.
